# EUROPÄISCHE PATENTSCHRIFT

(11) **EP 3 005 957 B1**
(45) Veröffentlichungstag und Bekanntmachung des Hinweises auf die Patenterteilung: **25.11.2020**
(21) Anmeldenummer: 15002466.9
(22) Anmeldetag: 20.08.2015
(51) Int. Cl.: A61B 17/12, A61B 17/122, A61B 17/28, A61B 17/128, A61B 17/29, A61B 17/00, A61B 18/14

(54) **CHIRURGISCHES INSTRUMENT**
SURGICAL INSTRUMENT
INSTRUMENT CHIRURGICAL

(30) Priorität: 09.10.2014 DE 102014014733
(43) Veröffentlichungstag der Anmeldung: 13.04.2016
(73) Patentinhaber: CARDIOMEDICAL GmbH, 30855 Langenhagen (DE)
(72) Erfinder: Wiedenbein, Wolfgang, 30926 Seelze (DE)

(56) Entgegenhaltungen:
- EP-A1- 1 878 390
- EP-A1- 2 335 609
- WO-A1-2008/115288
- WO-A1-2009/064807
- WO-A2-2013/003256
- US-A- 6 036 706

## Beschreibung

Die vorliegende Erfindung bezieht sich allgemein auf das Gebiet der Medizin, insbesondere der Chirurgie. Im chirurgischen Bereich werden Instrumente, Vorrichtungen oder Verfahren eingesetzt, um das Innere von lebenden Organismen zu untersuchen und/oder für operative Eingriffe zu nutzen. Zu den chirurgischen Instrumenten zählen alle medizinischen Instrumente, die vornehmlich in der Chirurgie Verwendung finden. Dazu gehören auch chirurgische Instrumente zum Abbinden oder anderweitigem Zusammendrücken von röhren-, bzw. schlauchförmigen Körperteilen, vorzugsweise von Blutgefäßen. Solche chirurgischen Instrumente werden dem Bereich der fassenden, bzw. klemmenden Instrumente zugeordnet und sind in einer großen Typenvielfalt vorhanden und hinreichend bekannt. Fassende, bzw. klemmende chirurgische Instrumente, werden beispielsweise bei Eingriffen in der Herz-, Thorax- und Gefäßchirurgie verwendet. In der Herz-und Thoraxchirurgie wird meistens eine offene Operation durchgeführt, bei der, durch die Eröffnung des Thorax, ein Zugang zum Herzen geschaffen wird. Der Zugang erfolgt in der Regel mittels einer medianen Sternotomie, wobei zum Öffnen des Brustkorbes ein, in etwa 25 cm langer Längsschnitt durch das Brustbein erzeugt wird. Bei der Thorakotomie erfolgt die chirurgische Öffnung des Thorax durch einen Interkostalschnitt, d.h. durch einen kleinen Schnitt in den Rippenzwischenraum. Die durch die Sternotomie oder Thorakotomie erzeugte Öffnung wird von einem Rippenspreizer, der zum Aufdehnen und Offenhalten des Brustkorbes eingesetzt wird, frei gehalten. Die Öffnung dient den Chirurgen als Zugang für operative Eingriffe. Die Eingriffe an den organischen Körperteilen erfolgen dann mit Hilfe einer Vielzahl unterschiedlicher chirurgischer Instrumente durch die erzeugte Öffnung im Brustkorb. Ist beispielsweise das Herz des Patienten freigelegt, werden direkt an das Herz und die großen Blutgefäße verschiedene Katheder, Kanülen und Klemmen angelegt. Typischerweise wird die Aorta mit einer Gefäßklemme rund um die aufsteigende Aorta okkludiert, um die Koronararterien vom Rest des Arteriensystems zu isolieren, wobei hier unter Okkludieren das Ergreifen, das Zusammendrücken, das Klemmen und Halten eines Gefäßes verstanden wird. Die notwendigerweise eingesetzten chirurgischen Instrumente verkleinern die Körperöffnung und behindern somit die Tätigkeit des Chirurgen in seinem Gesichtsfeld. Außerdem ist aufgrund der Größe der Öffnung, des entstandenen Gewebeschadens und des operativen Traumas, ein schneller Heilungsprozess bei dem Patienten nicht zu erwarten.

Die vorliegende Erfindung betrifft ein solches medizinisches Instrument, welches insbesondere in der Kardiologie als minimal-invasives chirurgisches Instrument eingesetzt werden kann, umfassend eine stufenlos fassende und klemmende Gefäßklemme, die zum Okkludieren von schlauchförmigen organischen, menschlichen oder tierischen Körperteilen, wie Blutgefäße, vorzugsweise Arterien u.dgl., für minimal-invasive chirurgische Eingriffe geeignet ist. Die Gefäßklemme umfasst ein Körperelement, ein Zug- und Druckelement und am distalen Ende ein Arbeitsmittel mit zwei Branchen, die, relativ zueinander, aus einer vollständig offenen bis in eine vollständig geschlossenen Stellung betätigbar sind, wobei die bewegbaren Branchen an einer vorderen- und hinteren Gelenkverbindung angeordnet sind, wobei die Gelenkverbindungen über eine vordere- und hintere Gelenkachse verfügen, die mit dem Körperelement verbunden sind. Am proximalen Ende ist eine Koppeleinrichtung, bestehend aus zwei Haltebacken, angeordnet. Die Koppeleinrichtung dient der Aufnahme einer lösbaren Zuführ-und Entnahmevorrichtung, die eine Greifeinrichtung, eine Bedieneinrichtung und ein Betätigungswerkzeug umfasst. Die Zuführ-und Entnahmevorrichtung ermöglicht das Einbringen und Entnehmen der Gefäßklemme durch eine Körperöffnung und ist durch einen Chirurgen bedienbar.

Des Weiteren befindet sich am proximalen Ende der Gefäßklemme eine Verstelleinrichtung in Kombination mit einer Arretierungseinrichtung, wobei die Verstelleinrichtung der Aufnahme eines lösbaren Betätigungswerkzeuges mit Drehgriff (Handhabungsteil mit Instrumentengriff) dient. Das Betätigungswerkzeug steht über eine Verstelleinrichtung mit einem Zug- und Druckelement in Verbindung und ermöglicht das Öffnen und Schließen eines Arbeitsmittels. Das Arbeitsmittel besteht aus parallel zueinander beweglichen Branchen, die stufenlos verstellt werden können.

Bei einem solchen medizinischen Instrument handelt es sich um ein, in drei Bauteile zerlegbares minimal-invasives chirurgisches Instrument, welches einen freien Zugang für andere Instrumente durch die Körperöffnung ermöglicht und somit die Instrumentenmenge in der Öffnung verringert. Das in drei Bauteile zerlegbare chirurgische Instrument umfasst eine Gefäßklemme (Arbeitsinstrument) und eine Zuführ- und Entnahmevorrichtung (Trägerinstrument bzw. Schaftteil), welche aus einer Bedieneinrichtung und einem Betätigungswerkzeug (Handhabungsteil nebst Instrumentengriff) besteht.

Medizinische Instrumente dieser Art sind aus dem Stand der Technik bekannt. Insbesondere die fassenden und klemmenden chirurgischen Instrumente in den verschiedenen Bauarten und Ausführungen, haben sich als chirurgische Instrumente bei operativen Eingriffen vielfach bewährt. Diese Instrumente haben den Vorteil, dass die Maulteile bzw. die Branchen den Schließdruck in jeder beliebigen Stellung über die Breite der zu klemmenden Gefäße, gleichmäßig verteilen.

Bereits die DE 7139469 zeigt eine Gefäßklemme mit zwei, um eine Achse schwenkbeweglich miteinander verbundenen, Branchenteilen. Diese Branchenteile sind durch Schwenkbewegungen, zum Schließen und Öffnen des Maules, in einander zugewandter bzw. abgewandter Richtung bewegbar. Durch die Verbindung der Maulteile mit den Branchenteilen über eine bekannte Parallelführung wird erreicht, dass die Maulteile bei der Bewegung der Branchenteile in einander zu- oder abgewandter Richtung, immer parallel zueinander bewegt werden, wobei die Maulteile lösbar mit den Branchenteilen verbunden sind. Die Parallelführung ist derart ausgebildet, dass jedes Maulteil mit einem Branchenteil um eine auf der den Griffen der Branchenteile abgewandten Seite der Schwenkachse der Branchenteile und in Abstand von dieser liegenden Achse schwenkbar und mit dem anderen Branchenteil auf der den Griffen der Branchenteile zugewandten Seite der Schwenkachse der Branchenteile und in Abstand von dieser mittels eines in einem Schlitz geführten Zapfens verbunden ist. Bei dieser Gefäßklemme handelt es sich um eine sogenannte sich öffnende und schließende scherenförmige Klemmzange. Eine solche Klemmzange besteht prinzipiell aus drei Bereichen, einem Arbeitsmittel, einem Schaftteil und einem Handhabungsteil. Der Hebelmechanismus der Gefäßklemme besteht darin, werden die Branchenteile aufeinander zu oder voneinander weg bewegt, dann werden die Maulteile um eine der Untersetzung der Parallelführung entsprechende Weglänge aufeinander zu bewegt, wobei die Maulteile immer in ihrer zueinander parallelen Stellung verbleiben. Diese Ausführungsform von Gefäßklemme erfüllt aber nicht die, aus der minimal-invasiven Chirurgie an sie gestellten Anforderungen. Einerseits fehlt eine stufenlose Einstellung der Maulteile, wodurch ein gleichmäßiges Klemmen mit möglichst wenig Traumatisierung des Gewebes ermöglicht werden kann, und anderseits entspricht die Ausführungsform nicht der einer platzsparenden Bauart, die im Zugang zum Körperinneren ein operatives Eingreifen begünstigt. Eine Trennung der Gefäßklemme in mehrere Bauteile bzw. eine Zerlegbarkeit der Gefäßklemme zwischen den Maulteilen und den Branchenteilen während der Operation ist nicht möglich.

Eine Weiterentwicklung der vorgenannten Gefäßklemme, insbesondere für die Endoskopie, offenbart im Stand der Technik die DE 44 12 171 A1. Bei dieser Ausführungsform wird das bewegbare Maulteil über eine parallelogrammartige Gelenkverbindung mit dem Halter, der gabelförmig ausgeformt ist, verbunden. Beide Maulteile werden aus einer geöffneten und/oder geschlossenen Gebrauchslage heraus, parallel zur Längsachse des Halters, stufenlos bewegt. Ferner sind die vorderen Maulgelenke und hinteren Maulgelenke mit einem Halter verbunden. Dabei besitzen die vorderen Maulgelenke über eine vordere Gelenk- und Halteachse, nahe dem Ende des Halters, eine gemeinsame Achse, wobei sich beide vorderen Maulgelenke überlappen. Des Weiteren können die vorderen Maulgelenke beim Schließen der Maulteile in diese eintauchen. Die hinteren Maulgelenke sind an dem Halter gemeinsam über eine hintere Gelenkachse befestigt, wobei die Gelenkachse verschiebbar ist. Dabei ist die hintere Gelenkachse mit einer Öffnung zur Durchführung eines Zug- und Druckelementes versehen, wobei das hintere Maulgelenk mittels einer hinteren Maulgelenkachse in eine Nut eingreift. Ausgehend von einem Handgriff, verläuft durch ein hülsenförmiges Arbeitselement ein Zug- und Druckelement in den Halter und endet dort an einer Stangengelenkachse. Die Stangengelenkachse wird in einem in einem Schlitz des Halters geführt und ist über Zug- und Druckelemente mit den hebelförmigen vorderen Maulgelenken verbunden. Der Hebelmechanismus der Gefäßklemme besteht darin, dass die vorderen- und hinteren Maulgelenke parallelogrammartig in den Nuten der Maulteile und in der Nut des gabelförmigen Halters verschoben werden können, wobei die Maulgelenke einerseits, beim Schließen der Maulteile, zwischen die Gabeln des Halters und andererseits in die Maulteile eintauchen. Durch eine Bewegung des Zug- und Druckelementes erfolgt ein Verschieben der Stangengelenkachse in einen Schlitz des Halters, wodurch sich die Maulteile öffnen oder schließen. Die parallelogrammartige und stufenlose Verstellung dieser Gefäßklemme wird als naheliegender Stand der Technik angesehen. Nachteilig bei dieser Ausführungsform eines endoskopischen Instruments ist die nicht platzsparende Bauart, welche im Zugang zum Körperinneren kein operatives Eingreifen begünstigt und die Anzahl der, in der Körperöffnung liegenden, Instrumente nicht verringert. D.h., die Zerlegbarkeit des chirurgischen Instrumentes bei der Benutzung ist nicht gegeben, weil eine Trennung des Maulteiles (Arbeitsinstrument) vom Halter (Schaftteil und Handhabungsteil) während der Operation nicht möglich ist.

Die Zerlegbarkeit eines chirurgischen Instrumentes wird in der DE 197 19 090 A1 offenbart. Hierbei handelt es sich um ein minimal-invasives, zerlegbares chirurgisches Instrument mit, ein Arbeitsinstrument (Arbeitsmittel) bildenden Maulteilen (Branchen) zum Greifen, Halten und/oder Klemmen von Organen für chirurgische minimal-invasive Eingriffe, wobei die Maulteile, zum Schließen und Öffnen des Arbeitsinstrumentes, parallel aufeinander zu und voneinander weg bewegbar sind. Das parallele Aufeinander zu- und Voneinander weg- bewegen der Maulteile erfolgt über zwei Paare von jeweils eine Außenkurbel und eine Innenkurbel umfassenden Kurbeln, die ein antiparalleles Kurbelgetriebe in Form einer sogenannten Nürnberger Schere bilden. Eine Drehbewegung eines Schraubgetriebes wird durch eine translatorische Bewegung des Kurbelgetriebes umgesetzt, wobei zur Ausführung der Parallelverlagerung der Maulteile das Kurbelgetriebe oder eine Kulissenführung und ein, damit verbundenes Schub- und Zugelement, benutzt wird. Dieses Instrument ist während des Gebrauchs bzw. während der Operation zerlegbar. Zur Durchführung der Zerlegbarkeit umfassen das Arbeitsinstrument (Arbeitsmittel) und das Trägerinstrument (Schaftteil) Kupplungsmittel, die zusammenwirken, wobei die Kupplungsmittel eine, auch während der Instrumentenverwendung lösbare Kupplungsverbindung zwischen dem Arbeitsinstrument (Arbeitsmittel) und dem Trägerinstrument (Schaftteil), bilden. Die Kupplungsmittel umfassen einen Schiebeschaft und einen Instrumenteneinsatz, siehe hierzu Anspruch 11. Der Nachteil des Instrumentes ist beim Handling festzustellen. Der Nachteil besteht in der Kupplungsfähigkeit der Kupplungsmittel während des Gebrauchs des Arbeitsinstrumentes (Arbeitsmittel) dieses vom Instrumentenschaft (Trägerinstrument) zu trennen und wieder zusammenzuführen. Zur Herstellung der Verbindung zwischen dem Arbeitsinstrument und dem Instrumentenschaft bzw. Trägerinstrument müssen die Mittelachse des Arbeitsinstrumentes und die Mittelachse des Trägerinstrumentes miteinander fluchten. Dieses ist während des operativen Einsatzes so gut wie nie gegeben, weil die Gefäße relativ weich und beweglich sind, wodurch das Arbeitsinstrument und somit dessen Mittelachse eine Lage einnehmen, die nicht mit der Mittelachse des zugeführten Trägerinstrumentes übereinstimmt. Des Weiteren ist nachteilig, dass die Herstellung eines solchen Arbeitsinstrumentes, eines solchen Trägerinstrumentes und eines solchen Instrumentengriffes, aufgrund der technischen Komplexität sehr anspruchsvoll ist, wodurch die Herstellungskosten des gesamten Instruments zu hoch sind. Diese hohen Herstellungskosten sind durch die Vielzahl der benötigten Einzelteile des Instrumentes auch erklärbar. Ein weiterer Nachteil besteht darin, dass die Vielzahl von einzelnen kleinen Teilen dazu führen kann, dass die Klemme Hohlräume, Nischen usw. aufweist, die bei der Verwendung des Instrumentes bei einer Operation leicht verunreinigen können, wodurch die Funktion des Instrumentes beeinträchtigt und die Sicherheit nicht mehr gewährleistet werden kann.

Ein weiteres chirurgisches Klemmsystem mit einer Klemme, die zwei Maulteile aufweist, die sich gegenseitig parallel zueinander öffnen und schließen lassen und die relativ zueinander aus einer vollständig offenen bis in eine vollständig geschlossene Stellung betätigbar sind und über eine lösbare Zuführ- und Entnahmevorrichtung mit einer Bajonettverbindung verfügen, ist der DE 602 11 044 T2 und der DE 602 24 460 T2 zu entnehmen. Das Einbringen oder Entnehmen der Klemme aus dem Körperinneren erfolgt über ein Betätigungsorgan, das mit der Zuführ- und Entnahmevorrichtung zusammenwirkt, wobei die Klemme einen Schrauben-/Mutter-Antrieb oder einen Schneckentrieb aufweist, die mit dem proximalen Enden der Maulteile in Eingriff stehen. Beim Schrauben-/Mutter-Antrieb ist die Schraube permanent mit einer Schub-Zug-Stange gekoppelt, die mit dem Maulteil, mittels eines Gelenkes, in Eingriff steht. Ein Drehen der Mutter bewirkt eine axiale Verschiebung der Schraube, welche die Schub-Zug-Stange schiebt oder zieht. Ist die Mutter durch ein Schneckentrieb und die Schraube durch eine Zahnstange ersetzt, handelt es sich beim Getriebesystem um ein Schneckengetriebe. Auch bei diesen Instrumenten sind, wie bei dem vorgenannten Instrument aus der DE 197 19 090 A1, die Kupplungsfähigkeit der Kupplungsmittel während des Gebrauchs ein Arbeitsinstrument vom Instrumentenschaft zu trennen und wieder mit diesem aufzunehmen mit wesentlichen Nachteilen versehen. Die Nachteile bestehen im Handling des Instrumentes, die sich aus der technischen Ausführungsform der Bajonettverbindung ergeben. Zur Herstellung der Verbindung zwischen dem Arbeitsinstrument und dem Instrumentenschaft bzw. Trägerinstrument, müssen die Mittelachse des Arbeitsinstrumentes und die Mittelachse des Trägerinstrumentes miteinander fluchten. Dieses ist während des operativen Einsatzes und bei der Entnahme aber nicht gegeben, weil die im Körperinneren eingesetzte Klemme eine Lage einnimmt, bei der die Mittelachse des Arbeitsinstrumentes nicht mit der Mittelachse des Trägerinstrumentes übereinstimmt. Wie kompliziert die Entnahme einer Klemme ist, ist aus der nachfolgenden Beschreibung ersichtlich. Wird die eingebrachte Klemme nicht mehr benötigt, kann die Zuführ- und Entnahmevorrichtung wieder in die Operationsstelle eingeführt werden, und unter Verwendung eines zusätzlichen Greifmittels wird die Klemme unbeweglich gehalten. Das distale Ende des hohlen Schaftes wird dann über das proximale Ende der Klemme gestreift, und der Handgriff wird gleichzeitig gedrückt und gedreht, damit die Bajonettstifte mit dem Bajonett in Eingriff kommen. Während der Handgriff gehalten wird, wird das Betätigungsorgan in das proximale Ende des Handgriffes eingeführt, und durch den hohlen Schaft hindurchgeführt und gleichzeitig in dem hohlen Schaft gedreht, bis das distale Ende in den Schlitz der Mutter eindringt und mit diesem in Eingriff kommt, zu welchem Zeitpunkt die Raste mit der Nut in Eingriff kommt und das Betätigungsorgan an Ort und Stelle verriegelt. Der Knopf wird dann gedreht, während der Handgriff unbeweglich gehalten wird, um das Maulteil der Klemme zu öffnen. Jetzt kann die Klemme vom Gewebe entfernt werden. Aus dieser Beschreibung ist das komplexe Handling ersichtlich, welches nötig ist, eine Klemme zu entfernen, und was nur mit einem zusätzlichen Instrument (Greifmittel) möglich ist, welches die Klemme unbeweglich hält, damit die Zuführ- und Entnahmevorrichtung mit der Klemme verbunden werden kann. Ein weiterer Nachteil der Klemme besteht darin, dass bei den kleinen Teilen des Schneckengetriebes es dazu führen kann, das die Hohlräume, Nischen, Verzahnungen usw. leicht Verunreinigen können, wodurch die Funktion des Instrumentes beeinträchtigt und die Sicherheit bei einer Verwendung des Instrumentes bei einer Operation nicht mehr gewährleistet werden kann. Ein weiterer Nachteil besteht in der technischen Ausführung der chirurgischen Klemme, die bei der Herstellung hohe Kosten verursacht.

Als nächstliegender Stand der Technik bei der stufenlosen Verstellung eines Maulteiles und der Zerlegbarkeit eines chirurgischen Instrumentes, wird die EP 2 335 609 A1 angesehen. Die EP 2 335 609 B1 offenbart ein zerleg- und komplettierbares chirurgisches Instrument. Das zerlegbare chirurgische Instrument umfasst eine Gefäßklemme, bestehend aus einem Arbeitsinstrument (Arbeitsmittel) mit zwei Branchen, eine Zuführ- und Entnahmevorrichtung als Trägerinstrument, welches aus einer Bedieneinrichtung und einem Betätigungswerkzeug (Handhabungsteil) mit Instrumentengriff besteht. Dieses chirurgische Instrument wird, aufgrund seiner einfachen Zerlegbarkeit während des Gebrauchs bzw. während der Operation in der minimal-invasiven Chirurgie eingesetzt. Die Gefäßklemme wird mit Hilfe einer einfach lösbaren Bedieneinrichtung erfasst, wobei ein ein- und ausschiebbares Betätigungswerkzeug die bewegliche Branche des Arbeitsmittels öffnen und schließen kann. Die Gefäßklemme enthält dazu eine Koppeleinrichtung und eine stufenlose Verstelleinrichtung. Zum Ergreifen bzw. Aufnehmen, sowie zum Lösen bzw. Trennen der Gefäßklemme von der Bedieneinrichtung (Trägerinstrument), enthält die Bedieneinrichtung eine Greifeinrichtung und weist eine Feststellvorrichtung auf, womit eine lösbare Verbindung zwischen der Gefäßklemme und der Bedieneinrichtung auf einfache Art und Weise hergestellt werden kann. Die Koppeleinrichtung und die Greifeinrichtung bilden die Kupplungsmittel, die zusammenwirken, wobei die Kupplungsmittel eine, auch während der Instrumentenverwendung lösbare Kupplungsverbindung zwischen dem Arbeitsinstrument (Arbeitsmittel) und der Bedieneinrichtung (dem Trägerinstrument) bilden. Das Betätigungswerkzeug (Handhabungsteil) mit Instrumentengriff greift, zum stufenlosen Öffnen und Schließen der Branche, in die Verstelleinrichtung der Gefäßklemme ein. Der Nachteil dieser Ausführungsform des minimal-invasiven chirurgischen Instrumentes besteht darin, dass die zum Schließen und Öffnen des Arbeitsinstrumentes (Arbeitsmittels) bildenden Maulteile (Branchen) nicht parallel aufeinander zu und voneinander weg bewegbar sind, sondern nach dem Funktionsprinzip einer Schere arbeiten, wodurch ein gleichmäßiges Ergreifen, Halten und/oder Klemmen von Organen für chirurgische minimal-invasive Eingriffe, nicht möglich ist.

Als nächstliegender Stand der Technik bei Klemmwerkzeugen mit parallel klemmendem Maulteil wird die WO2009/064807 A1 angesehen, die ein Klemmsystem und ein Verfahren zum Benutzen eines Klemmwerkzeuges aufzeigt. Gezeigt wird ein Klemmwerkzeug, welches als medizinisches bzw. chirurgisches Instrument z.B. für die Kardiologie als minimalinvasive, stufenlos fassende- und klemmende Gefäßklemme, eingesetzt werden kann. Das Klemmwerkzeug weist ein Handstück auf, das einen feststehenden und einen beweglichen Handgriff mit Fingerösen hat, ein Außenrohr, das mit dem feststehenden Handgriff verbunden ist und ein, in dem Außenrohr verschiebbar geführtes Zugelement, in Form einer Zugstange. Die Zugstange ist einerseits mit den Klemmelementen der Klemme und andererseits mit dem Drehknopf am beweglichen Handgriff des Klemmwerkzeuges verbunden. Eine Zerlegung und Zusammensetzung des Klemmwerkzeuges ist möglich. I.d.r. erfolgt eine Zerlegung und Zusammensetzung eines Zugelementes bzw. einer Zugstange durch technische Mittel, wie z.B. von Bajonettverschlüssen bekannt. Mit einem Bajonettverschluss würde eine Trennung und Zusammenfügung zwischen einer Klemme und einem Klemmwerkzeug ermöglicht. Aus den Figuren 18 bis 22 der WO 2009/064807 A1 ist ersichtlich, dass eine Trennstelle im Klemmwerkzeug zwischen der Hülse des Außenrohres eines feststehenden Handgriffes und dem Klemmkörper der Klemme vorgesehen ist. Bei einer bestimmten Stellung des feststehenden Handgriffes zwischen einem Marker und einem Endstück, die auf einem Positionierzeiger bzw. einem Rastarm angeordnet sind, kann eine Trennstelle zwischen der Hülse und dem Klemmkörper, freigegeben werden. Die Freigabe erfolgt dann durch Verschieben einer Zugstange, die mit dem beweglichen Handgriff verbunden ist. Nach der Freigabe kann der, vom beweglichen Handgriff kommende Anschlusshaken der Zugstange, aus der Öffnung der, von der Klemme kommenden Anschlussverbindungsstange, ausgehängt werden. Der Nachteil der Zerlegbarkeit und Zusammensetzung dieser Gefäßklemme besteht darin, dass eine Zerlegung und Zusammensetzung des Instruments nicht während der Operation bzw. wenn die Gefäßklemme im Körper eines Patienten eingeführt ist, erfolgen kann. Das sichere Ergreifen der Gefäßklemme während der Operation durch eine Bedieneinrichtung ist auch nicht möglich. Weiter nachteilig ist, das bei der Zerlegung der vorliegenden Ausführung von Klemmwerkzeug in die beiden Bestandteile, Klemmwerkzeug und Klemme, die Klemmbacken der Klemme nach der Zerlegung keinen Druck mehr auf ein Gefäß mehr ausüben können, weil die Verbindung der Zugstange der Klemme, mit der Zugstange zum beweglichen Handgriff des Klemmwerkzeuges, unterbrochen ist. Auf die Klemmbacken wirkt durch die Trennung der Zugstangen keine Kraft mehr ein, wodurch die Klemmung eines Gefäßes nicht mehr möglich ist. Ein dosiertes Klemmen von Gefäßen nach der Trennung des Klemmwerkzeuges von der Klemme ist unmöglich und die Klemmbacken können nicht in einer x-beliegen Klemmstellung arretiert werden.

Oft besteht die Anforderung, ein Gefäß mit parallel sich öffnenden und schließenden Klemmbacken zu klemmen. Dieser Anforderung kommt die WO 2009/064807 A1, wie in den Figuren 9a und 9b gezeigt, nach. Aus den Figuren 9a und 9b ist eine Ausführungsform eines Klemmwerkzeuges mit parallel sich öffnenden und schließenden Klemmbacken ersichtlich.

Die Klemme des Klemmwerkzeuges umfasst ein Körperelement, zwei Schub-Zug-Stangen als Zug- u. Druckelement, vier Hebelarme bzw. vier Stangen und zwei Klemmbacken. Die Klemmbacken bilden das distale Ende des Körperelementes und sind relativ zueinander aus einer vollständig offenen bis in eine vollständig geschlossene Stellung betätigbar, wobei die bewegbaren Klemmbacken über eine vordere und hintere parallelogrammartige Gelenkverbindung, die über eine vordere und hintere Gelenkachse verfügen, mit dem Körperelement verbunden sind.

Die Stangen (Hebelarme) bilden als Viergelenkgetriebe die mechanische Verbindung zwischen den Klemmbacken und dem Körperelement der Klemme, wobei die Klemme prinzipiell ein Hebelgetriebe umfasst, welches aus einem feststehenden Hebel besteht, der als Körperelement ausgebildet ist und der zwei unabhängig voneinander annähernd axial verschiebbare Schub--Stangen umfasst, die längs der Mittellinie des Körperelementes, sowie oberhalb und unterhalb des Körperelementes und durch dieses parallel beabstandet, angeordnet sind. Des Weiteren wird das Hebelgetriebe aus sechs drehbeweglichen Hebeln gebildet, die aus zwei parallel beabstandeten Klemmbacken, einem ersten Paar von Hebeln und einem zweiten Paar von Hebeln gebildet werden. Die beiden Schub-Zug-Stangen sind als Zug- und Druckelement ausgebildet und an vier drehbeweglichen Hebelarmen bzw. vier Stangen angeordnet, aber nicht am Körperelement. Des Weiteren verschieben die beiden Schub-Zug-Stangen die vier Stangen (Hebelarme) um die vier Drehpunkte im Körperelement. Die beiden Schub-Zug-Stangen verschieben die vier Stangen (Hebelarme) um die vier Drehpunkte im Körperelement. An vier weiteren Drehpunkten, die am anderen Ende der vier Stangen (Hebelarmen) liegen, sind zwei parallel beabstandete Klemmbacken ausgebildet, wobei die Klemmbacken längs und parallel zur Mittellinie des Körperelementes beabstandet sind und über die vier Stangen (Hebelarme) mit dem Körperelement drehbeweglich am freien Ende des Körperelementes verbunden sind. Der Nachteil dieses Hebelgetriebes besteht darin, dass jede Klemmbacke einen separaten Antrieb über eine Schub-Zug-Stange aufweist und somit zwei Antriebe für das Hebelgetriebe vorgehalten werden müssen, wodurch Fehler beim Schließen der Klemmbacken auftreten können und es bei einem abzuklemmenden Gefäß zu einem Schaden bzw. zur Traumatisierung des Gewebes kommen kann.

Das Körperelement der Klemme weist am proximalen Ende eine Koppeleinrichtung bzw. einen Verbindungsanschluss auf, an welchem das Klemmwerkzeug angeschlossen oder getrennt werden kann. Der Verbindungsanschluss der Klemme mit den parallel beabstandeten Klemmbacken ist in den Figuren 9a, 9b aber nicht dargestellt.

Der Fachmann kann der WO 2009/064807 A1 nur entnehmen, dass zwei Schub-Zug-Stangen am Körperelement anliegen, wenn die Klemmbacken der Klemme geschlossen sind. Bei geöffneten Klemmbacken sind die Schub-Zug-Stangen zwangsläufig vom Körperelement beabstandet. Jede der beiden Schub-Zug-Stangen ist gleichzeitig an zwei Hebelarmen bzw. Stangen und an jeder Stange über einen Drehpunkt angeordnet, wodurch vier Drehpunkte für die parallele Verschiebung der Klemmbacken vorhanden sind. Wie der Verbindungsanschluss der parallel geführten Klemmbacken an die, in der Hülse des Klemmwerkzeuges geführte Schub-Zug-Stange erfolgt und wie das Körperelement der Klemme mit der Hülse des Klemmwerkzeuges korrespondiert, ist nicht ersichtlich. Die Koppeleinrichtung der WO 2009/064807 A1 für ein nicht parallel klemmendes Maulteil kann den Figuren 8a - 8c und 13 entnommen werden und besteht aus einem Kegel bzw. einem ringförmigen, konischen Anlageelement, der im Körperelement angeordnet ist und der in einen korrespondierenden Ringsitz der Hülse des Klemmwerkzeuges eingreifen kann. Des Weiteren weist die Schub-Zug-Stange des Klemmwerkzeuges einen Haken auf, der in eine Öffnung der Schub-Zug-Stange der Klemme eingreifen kann. Eine technische Lösung für die Kopplung von zwei im Körperelement und in der Hülse bis zum Drehknopf des Klemmwerkzeuges geführten Schub-Zug-Stangen, ist nicht angegeben. Ein Nachteil dieser Ausführungsform einer parallelen Klemme, gemäß der Figuren 9a, 9b ist, dass die Koppelstelle an der Klemme keine Mittel aufweist, mit welcher die Stellung der Klemmbacken, wenn diese vom Klemmwerkzeug getrennt sind, fest fixiert werden kann.

Als nächstliegender Stand der Technik bei Gefäßklemmen mit parallel schließendem Maulteil wird die WO2008/115288 A1 angesehen. Die WO2008/115288 A1 offenbart ein chirurg. Instrument, welches den Rohrschaft-Instrumenten zugeordnet wird. Bei dem chirurg. Instrument handelt es sich um eine fassende Klemme. Die Klemme weist am proximalen Ende eine pistolengriffartige Betätigung auf. Am distalen Ende der Klemme ist ein parallel öffnendes und schließendes Maulteil angeordnet.
Oft besteht die Anforderung, ein Gefäß mit zwei sich parallel öffnenden und schließenden Klemmbacken zu klemmen. Dieser Anforderung kommt die WO 2008/115288 A1, wie z.B. in den Figuren 1B, 2B, 28 und 30 gezeigt, nach, aus denen eine Ausführungsform eines Klemmwerkzeuges mit zwei sich parallel öffnenden und schließenden Klemmbacken ersichtlich ist.
Diese Ausführungsform von zwei sich parallel öffnenden und schließenden Maulteilen wird als Gefäßklemme verwendet. Die Verbindung zwischen der pistolengriffartigen Anordnung und der Gefäßklemme erfolgt über einen Rohrschaft. In dem Rohrschaft wird eine Betätigungsstange geführt, die das Öffnen und Schließen des parallelen Maulteils der Gefäßklemme ermöglicht. Dazu ist die, im Rohr verschiebbare Betätigungsstange an einem, im Rohr geführten Zug-Druckelement angeschlossen. Das, der Betätigungsstange gegenüberliegende Ende des Zug-Druckelementes weist eine aus zwei Fingern bestehende Halterung auf. An den Fingern ist jeweils ein Stift angeordnet. Die beiden Stifte sind in senkrechter Richtung zur Mittellinie des Rohrschaftes zueinander beabstandet und greifen jeweils in einen geöffneten Schlitz eines ersten Schwenkarmes (drive element) ein. Die beiden ersten Schwenkarme (drive element) sind in einem Gehäuse, welches sich an den Rohrschaft anschließt, angeordnet. Zur Befestigung der beiden ersten Schwenkarme (drive element) in dem Gehäuse, ist das Gehäuse vom Ende her geöffnet. In diese Öffnung greift das eine Ende der beiden ersten Schwenkarme (drive element) ein, wobei diese auf einer gemeinsamen Achse im Gehäuse drehbeweglich angeordnet sind. Die gemeinsame Achse der ersten Schwenkarme (drive element) liegt auf der Mittellinie des Rohrschaftes. Das andere Ende der beiden ersten Schwenkarme (drive element) weist ebenfalls eine Bohrung auf. Die Bohrung dient der Aufnahme einer Achse, wobei die Achse gleichzeitig in die Bohrung einer Klemmbacke eingreift.

Die beiden Klemmbacken bilden das sich parallel öffnende und schließende Maulteil der Gefäßklemme. Die Klemmbacken sind daher mit einer innen liegenden Verzahnung, in Richtung des Gefäßes zeigend, versehen. Die beiden Klemmbacken weisen unmittelbar neben der Achse für den Anschluss eines Schwenkarmes (drive element) eine weitere Bohrung auf. Diese Bohrung dient über eine weitere Achse wiederum dem Anschluss eines zweiten Schwenkarmes (second jaw). Die Bohrung zur Aufnahme der Achse für den ersten Schwenkarm (drive element) und die Bohrung zur Aufnahme der Achse für den zweiten Schwenkarm (second jaw) sind unmittelbar übereinander und nicht hintereinander in den Klemmbacken angeordnet. D.h., die Mittellinie der Bohrung für die Achse des ersten Schwenkarmes (drive element) und die Mittellinie der Bohrung für die Achse des zweiten Schwenkarmes (second jaw) sind senkrecht, in Längsrichtung der Branche betrachtet und parallel übereinander, beabstandet. Zur Aufnahme des ersten Schwenkarmes (drive element) und des zweiten Schwenkarmes (second jaw) an der Klemmbacke weist diese zwei Öffnungen, die durch einen Mittelsteg beabstandet sind, auf. Der Mittelsteg enthält sowohl die Bohrung für die Achse des ersten Schwenkarmes (drive element) als auch für die Bohrung für die Achse des zweiten Schwenkarmes (second jaw). Das andere Ende der zweiten Schwenkarme (second jaw) ist ebenfalls, wie die ersten Schwenkarme (drive element), in der gleichen Öffnung des Gehäuses angeordnet. Die beiden zweiten Schwenkarme (second jaw) befinden sich am freien Ende des Gehäuses auf einer gemeinsamen Achse. Die Achse für die zweiten Schwenkarme (second jaw) befindet sich ebenfalls auf der Mittellinie des Rohrschaftes. Die beiden, auf der Mittellinie des Rohrschaftes liegenden Achsen der ersten und zweiten Schwenkarme, sind beabstandet. Die Achse für die zweiten Schwenkarme (second jaw) befinden sich am freien Ende des Gehäuses und die Achse der ersten Schwenkarme (drive element) befinden sich beabstandet zur ersten Achse annähernd zwischen den, an den Fingern angeordneten Stiften des Zug-Druckelements. Die zweiten Schwenkarme (second jaw) weisen eine "U"-förmige Ausführungsform auf. Die Außenseite des "U"-förmigen Querschenkels ist Träger der Verzahnung, in Richtung des Gefäßes zeigend, und die Innenseite des "U"-förmigen Querschenkels bildet die Anlagefläche für den ersten Schwenkarm (drive element), wenn das Maulteil der Gefäßklemme geschlossen wird. Beim Schließen des parallel geführten Maulteils, fahren die ersten Schwenkarme (drive element) zwischen die Schenkel der "U"-förmigen Stege und kommen auf der Rückseite der zweiten Schwenkarme (second jaw) zum Anliegen.

Die Klemmbacken des Maulteils bilden das distale Ende der Gefäßklemme und sind relativ zueinander aus einer vollständig offenen bis in eine vollständig geschlossene Stellung betätigbar. Um ein paralleles Klemmen der verstellbaren Klemmbacken zu ermöglichen, sind daher zwei Gelenkverbindungen, eine vordere- und eine hintere Gelenkverbindung, an den Klemmbranchen, vorgesehen. Die vordere Gelenkverbindung besteht aus zwei gleichen Schwenkarmen (second jaw) und weist drei Gelenkachsen auf. Die hintere Gelenkverbindung besteht ebenfalls aus zwei gleichen Schwenkarmen (drive element) und weist ebenfalls drei Gelenkachsen auf, wobei die vorderen Schwenkarme (second jaw) und die hinteren Schwenkarme (drive element) sich in ihrer Ausführungsform unterscheiden. Beide Gelenkverbindungen sind einerseits mit dem Gehäuse und andererseits mit den Klemmbacken verbunden. Die an den Klemmbacken angeordneten Gelenkachsen der vorderen- und hinteren Schwenkarme, sind senkrecht zur Mittellinie des Rohrschaftes und unmittelbar in senkrechter Richtung nebeneinander angeordnet.

Die vordere- und die hintere Gelenkverbindung liegen im geschlossenen Zustand des Maulteils parallel aufeinander, s. Fig.27. Im geöffneten Zustand des Maulteils hingegen, s. z.B. Fig.28, bildet jeweils ein vorderer Schwenkarm (second jaw) mit einem hinterem Schwenkarm (drive element) und dem Abstand zwischen der vorderen Gelenkachse des Schwenkarms (second jaw) und der hinteren Gelenkachse des Schwenkarms (drive element) auf der Mittellinie des Rohrschaftes, ein stumpfwinkliges Dreieck. Die gemeinsame Gelenkachse der hinteren Schwenkarme (drive element) im Gehäuse bildet den Eckpunkt A und die gemeinsame Gelenkachse der vorderen Schwenkarme (second jaw) im Gehäuse bildet den Eckpunkt B des stumpfwinkligen Dreiecks, wobei beide Eckpunkte auf der Mittellinie des Gehäuses und des Rohrschaftes liegen. Annähernd im Eckpunkt C des stumpfwinkligen Dreiecks befinden sich die Gelenkachsen für die Verbindung zwischen den Klemmbacken und den vorderen- und hinteren Schwenkarmen. Beim Schließen der Klemmbacken geht im Eckpunkt A der Winkel α zwischen dem Schenkel (hinterer Schwenkarm) und der Verbindungslinie zwischen den Eckpunkten A-B gegen Null. Auch der Winkel β im Eckpunkt C zwischen dem Schenkel des hinteren Schwenkarms und dem vorderen Schenkel des Schwenkarms geht beim Schließen der Klemmbacken gegen Null. Nur der Winkel γ im Eckpunkt B zwischen der Verbindungslinie A-B und dem Schenkel des vorderen Schwenkarmes vergrößert sich und geht gegen 180 Grad.
Aufgrund der Anordnung der Schwenkarme am Gehäuse und an den Klemmbacken, sind die vorderen- und hinteren Schwenkarme nicht parallel zueinander beabstandet und können sich nicht parallel aufeinander zu oder voneinander weg bewegen.

Die Schwenkarme bilden als Hebel die mechanische Verbindung zwischen den Klemmbacken und dem Gehäuse der Gefäßklemme, wobei die Gefäßklemme prinzipiell ein Hebelgetriebe umfasst, welches aus einem Rohrschaft mit Gehäuse besteht, indem eine axial verschiebbare Betätigungsstange auf gleicher Mittellinie ein Zug-Druckelement verschiebt. Des Weiteren wird das Hebelgetriebe aus sechs drehbeweglichen Hebeln gebildet, die aus zwei parallel beabstandeten Klemmbacken, aus zwei vorderen Schwenkarmen und zwei hinteren Schwenkarmen gebildet werden. Am Zug-Druckelement sind die beiden hinteren Schwenkarme drehbeweglich angeordnet. Aufgrund der Verschiebung des Zug-Druckelements, werden die hinteren Schwenkarme um den Winkel α verschoben, wodurch, aufgrund der Verbindung der vorderen und hinteren Schwenkarme im Eckpunkt C, auch die vorderen Schwenkarme um den Winkel β verschoben werden. Die Verschiebung der vier Schwenkarme um die Winkel α, β, γ ist nur soweit möglich, bis der Winkel α der hinteren Schwenkarme gegen Null gegangen ist. Aufgrund der Anordnung der Klemmbacken im Eckpunkt C an den vorderen- und hinteren Schwenkarmen, verschieben sich die Klemmbacken auf einer Kreisbahn, bis die Klemmbacken aufeinander zu liegen kommen.

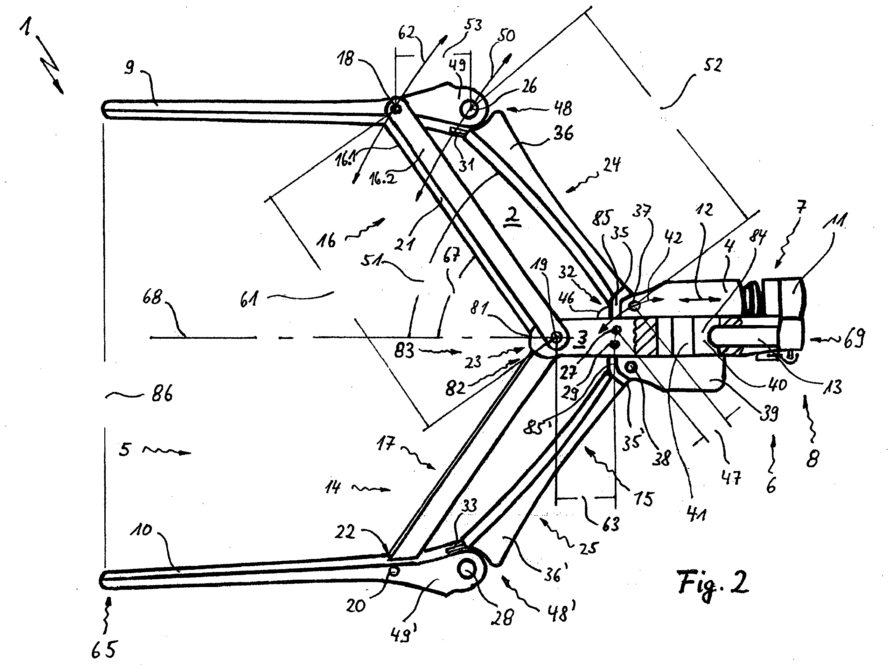

Die zuvor beschriebene Ausführungsform der Gefäßklemme mit parallel schließendem Maulteil ist vor allem den Figuren 2B, 26-31 zu entnehmen. Der Fachmann kann daraus eine Anordnung von vier Schwenkarmen entnehmen, die es den daran angeordneten Branchen ermöglichen sich beim Öffnen und Schließen parallel aufeinander zu und voneinander weg zu bewegen, wodurch ein Wegrollen eines eingeklemmten Gefäßes vermieden werden kann.

Um die, aus der minimal-invasiven Chirurgie gestellten Anforderungen an die chirurgischen Instrumente zu erfüllen, ist es notwendig, neue Ausführungsformen von chirurgischen Instrumenten zu entwickeln.

Gewünscht ist ein medizinisches Instrument der eingangs genannten Art für den Einsatz in der minimal-invasiven Chirurgie zu schaffen, welches die vorgenannten Nachteile und Unzulänglichkeiten der bekannten Anordnungen vermeidet. Entsprechend ist es eine Aufgabe der vorliegenden Erfindung, für eine chirurgische Gefäßklemme eine technische Lösung bereitzustellen, die einerseits einfach und kostengünstig in der Herstellung ist, und andererseits es auch ermöglicht, ein, in ergonomischer- und Handlings-Hinsicht mit einfacher Funktionsgeometrie, ausgestattetes chirurgisches Instrument für die erhöhten Ansprüche herzustellen. Diese chirurgische Gefäßklemme soll nicht nur organische Körperteile besonders gleichmäßig erfassen und klemmen, sondern es soll dem Chirurgen auch die Möglichkeit gegeben werden, eine unterschiedliche Klemmkraft einstellen zu können. Die unterschiedlich einstellbare Klemmkraft soll diversen medizinischen Einsätzen entsprechen. Ein solches minimal-invasives chirurgisches Instrument wird auch als MIC-Instrument bezeichnet und soll einige positive Eigenschaften der bekannten chirurgischen Klemmen aus dem Stand der Technik, aufweisen.

Eine weitere Aufgabe besteht darin, die Eigenschaften der einfachen Zerlegbarkeit eines Instrumentes, sowie das sichere Ergreifen eines Arbeitsinstrumentes (Arbeitsmittels) durch eine Bedieneinrichtung (Trägerinstrument), wie aus der EP 2 335 609 B1 bekannt, zu ermöglichen Des Weiteren sollen beide Maulteile, nachstehend als Branchen bezeichnet, am Arbeitsinstrument (Arbeitsmittel), nachstehend als Gefäßklemme bezeichnet, stufenlos verstell- und arretierbar sein. Die beiden Branchen der Gefäßklemme sollen, parallel zueinander, wie z.B. aus der DE 44 12 171 A1, der DE 197 19 090 A1 und der DE 602 24 460 T2 bekannt, zum Öffnen und zu Schließen eines Arbeitsmittels geeignet sein und ein gleichmäßiges Klemmen mit möglichst wenig Traumatisierung des Gewebes ermöglichen. Außerdem sollen sie in jeder beliebigen Stellung zwischen der vollständig offenen und der vollständig geschlossenen Stellung im Wesentlichen parallel zueinander verlaufen.

Als weitere Aufgabe soll, um die technischen Erfordernisse der Zerlegbarkeit und der parallelen Verstellung von Branchen, zum Öffnen und Schließen eines Arbeitsmittels zu erfüllen, ein Hebelgetriebe verwendet werden. Auch unter dem Gesichtspunkt einer Reinigung und Desinfizierung ist ein Instrument zu betrachten. Daher spielen das Design und die Reduzierung der Vielzahl von einzelnen Bauteilen bei einem Instrument eine wichtige Rolle. Ein solches zerlegbares, chirurgisches Instrument würde ein vorteilhaftes Handling aufweisen und, aufgrund seiner Zerlegbarkeit, das Operationsfeld bzw. das Gesichtsfeld des Operateurs und somit die Zugangsöffnung im Thorax nicht beeinträchtigen. Zugangsöffnungen haben typischerweise kleinstmögliche Durchmesser, was der Philosophie der minimal-invasiven Chirurgie entspricht. Aufgrund dieser Vorgaben sollen, soweit wie möglich, keine anderen Instrumente die Zugangsöffnung verkleinern. Das kann aber nur gewährleistet werden, wenn die Instrumente zerlegbar sind.

Um ein, mit diesen Merkmalen der vorliegenden Erfindung ausgestattetes chirurgisches Instrument herzustellen, insbesondere eine minimal-invasive chirurgische Gefäßklemme, zur Verwendung bei operativen Eingriffen am menschlichen oder tierischen Körper in der minimal-invasive Chirurgie, wird erfindungsgemäß vorgeschlagen, das chirurgische Instrument derart zu gestalten, dass dem Chirurgen eine ergonomisch gestaltete Zuführ- und Entnahmevorrichtung, bestehend aus einer Bedieneinrichtung und einem Betätigungswerkzeug mit Instrumentengriff zur Verstellung einer Gefäßklemme, für ein vereinfachtes Handling, zur Verfügung steht. Bei der Benutzung eines solchen chirurgischen Instruments kommt insbesondere der Bedienung (Handling) des Instrumentes eine besondere Bedeutung zu, um mögliche Verletzungen an Organen, Gefäßen und dgl. und/oder daraus entstehende traumatische Folgen zu vermeiden. Ein wichtiges Kriterium bei Operationen ist, die Anzahl von notwendigerweise benötigten Instrumenten und/oder die Baugröße der Instrumente, zu reduzieren. Eine Reduzierung einzelner chirurgischer Instrumente bei der Operation ist in der Regel sehr schwierig, so dass das Augenmerk auf die Verkleinerung der Baugröße von Instrumenten und die Zerlegbarkeit der Instrumente, gerichtet ist. Unter der Zerlegbarkeit wird hier das Zerlegen eines Instrumentes in verschiedene Komponenten verstanden, wobei die einzelnen Komponenten untereinander kompatibel sind und somit eine Reduzierung von chirurgischen Instrumenten ermöglichen. Um eine Kombinierbarkeit von einzelnen Komponenten zu erreichen, sind gleichwirkende Mittel bei den Kupplungselementen zu verwenden.

Entsprechend ist es eine Aufgabe der vorliegenden Erfindung, eine chirurgische Gefäßklemme herzustellen, die mit einer Koppeleinrichtung, einer Verstelleinrichtung in Kombination mit einer Arretierungseinrichtung, gemäß der EP 2 335 609 B1, ausgestattet ist, um eine Kompatibilität mit einer vorhandenen Zuführ- und Entnahmevorrichtung, bestehend aus einer Bedieneinrichtung und einem Betätigungswerkzeug zu erzielen. Das in drei Bauteile zerlegbare chirurgische Instrument aus der EP 2 335 609 B1 bildet die Basis der Weiterentwicklung. Die Zuführ- und Entnahmevorrichtung bzw. die beiden Bauteile, die Bedieneinrichtung und das Betätigungswerkzeug mit Instrumentengriff, sollen auch für andere, unterschiedlich ausgebildete Gefäßklemmen einsetzbar und somit vielfach wieder verwendbar sein, um das Durcheinander von Instrumenten an der Operationsstelle zu verringern. D.h., die Zuführ- und Entnahmevorrichtung soll für verschiedene Ausführungsformen von chirurgischen Instrumenten verwendet werden, insofern diese mit korrespondierenden Kupplungsmitteln ausgestattet sind.

Eine weitere Aufgabe der Erfindung ist es, die in der Ausführungsform unterschiedlich ausgebildeten Gefäßklemmen mit Kupplungsmitteln gemäß der EP 2 335 609 B1 zu versehen, die mit der Zuführ- und Entnahmevorrichtung kompatibel sind und welche die Handhabung des chirurgischen Instruments, insbesondere der erfinderischen Gefäßklemme, wesentlich erleichtern und somit sicherer gestalten und Kupplungsmittel z.B. Bajonettverschlüsse, wie aus der DE 602 24 460 T2 und DE 197 19 090 A1 bekannt, zu vermeiden.

Die erfinderische Gefäßklemme weist daher eine Koppeleinrichtung, eine Verstelleinrichtung und eine Arretierungseinrichtung, gemäß der EP 2 335 609 B1, auf. Die Haltebacken der Koppeleinrichtung an der Gefäßklemme korrespondieren dabei mit den Greifelementen der Greifeinrichtung an der Bedieneinrichtung bzw. an dem Trägerinstrument. Des Weiteren korrespondiert das an der Gefäßklemme angeordnete Verstellelement der Verstelleinrichtung mit der am Betätigungswerkzeug angeordneten Werkzeugklinge, und die Arretierungseinrichtung korrespondiert mit der Verstelleinrichtung. Die erfinderische Gefäßklemme muss derart ausgebildet sein, dass die Zuführ- und Entnahmevorrichtung die Gefäßklemme erfassen, klemmen, führen und entsprechend wieder freigeben kann, wobei das Betätigungswerkzeug die beweglichen Branchen des Arbeitsmittels stufenlos über die Verstelleinrichtung, in Verbindung mit dem Zug- und Druckelement, verstellen kann. Das Arretieren der verstellbaren Branchen erfolgt über eine Arretierungseinrichtung. Zur vorteilhaften Ausführung eines kompletten chirurgischen Instrumentes wird auf die ausführlichen Beschreibungen in der EP 2 335 609 B1 verwiesen. Zur Lösung der Aufgabe wird vorgeschlagen, dass das feststehende Körperelement der Gefäßklemme daher mit einer Koppeleinrichtung ausgestattet ist, die mit einer Zuführ- und Entnahmevorrichtung aus der EP 2 335 609 B1 korrespondiert.

Eine weitere Aufgabe besteht darin, eine mit einer Zuführ- und Entnahmevorrichtung bedienbaren Gefäßklemme mit zwei parallel sich öffnenden und schließenden Branchen auszubilden. Zur Lösung dieser Aufgabe führt, dass die bewegbaren Branchen über eine vordere- und hintere Gelenkverbindung mit dem Körperelement verbunden sind, wobei die Gelenkverbindungen mit dem Körperelement ein Hebelgetriebe bilden.

Das aus den zu lösenden Aufgaben und in der Entwicklung entstandene chirurgische Instrument, insbesondere in der Ausführung eines parallel geführten Arbeitsmittels, bestehend aus zwei Branchen an einer Gefäßklemme, wurde erfindungsgemäß, um die Öffnung im Thorax von dem bei der Operation benötigten chirurgischen Instrument frei zu halten, an die dreiteilig zerleg- und komplettierbaren Instrumente angepasst. Die dreiteilig zerleg- und komplettierbaren Bauteile des chirurgischen Instruments betreffen eine Bedieneinrichtung, ein Betätigungswerkzeug und, wie nachstehend beschrieben, eine erfinderische Gefäßklemme. D.h., die Lösung der Aufgabe besteht darin, eine Gefäßklemme mit stufenlos parallel verstellbaren Branchen herzustellen, die an das Handling eines zerleg- und komplettierbaren chirurgischen Instrumentes angepasst ist und mit den einzelnen Instrumententeilen eines anderen chirurgischen Instrumentes korrespondiert. Die Lösung findet sich in den erfinderischen Merkmalen, die in den Ansprüchen aufgeführt sind, wieder. Die vorteilhaften Ausführungen des erfinderischen chirurgischen Instrumentes können der nachstehenden Beschreibung entnommen werden.

Die Lösung dieser Aufgabe besteht darin, eine stufenlos fassende- und klemmende Gefäßklemme mit zwei sich parallel öffnenden und schließenden Branchen, gemäß der Figur 2, zu entwickeln. Die Gefäßklemme ist im Prinzip ein technisches System, welches aus einer Vielzahl verschiedener technischer Komponenten, bzw. einzelner Elemente, zusammengefügt ist. Die einzelnen Komponenten bzw. Elemente der Gefäßklemme umfassen ein Körperelement, ein Zug- und Druckelement, am distalen Ende ein Arbeitsmittel, welches aus zwei Branchen besteht. Die zwei Branchen sind aus einer vollständig offenen bis in eine vollständig geschlossene Stellung relativ zueinander betätigbar, wobei die bewegbaren Branchen an einer vorderen- und hinteren Gelenkverbindung angeordnet sind. Die Gelenkverbindungen verfügen über eine vordere- und hintere Gelenkachse, die mit dem Körperelement verbunden sind. Am proximalen Ende der Gefäßklemme befindet sich eine Koppeleinrichtung, bestehend aus zwei Haltebacken. Die Haltebacken dienen der Aufnahme einer lösbaren Zuführ- und Entnahmevorrichtung, die durch den Chirurgen bedienbar ist. Des Weiteren weist die Gefäßklemme eine Verstelleinrichtung in Kombination mit einer Arretierungseinrichtung auf, wobei die Verstelleinrichtung zur Aufnahme eines lösbaren Betätigungswerkzeuges dient. Das Betätigungswerkzeug in Verbindung mit der Verstelleinrichtung, nimmt über das Zug- und Druckelement eine stufenlose Verstellung der beiden Branchen vor, wodurch ein Öffnen und Schließen der des Arbeitsmittels erfolgt.

Das Herzstück der Erfindung bei der Gefäßklemme umfasst ein Hebelgetriebe, bestehend aus einer Hebelmechanik. Das Wirkprinzip des Hebelgetriebes ist im Grunde ein technologischer Vorgang, bei dem das Zusammenwirken der physikalischen und geometrischen Vorgänge, im Zusammenspiel mit den vorgenannten Komponenten bzw. Elementen, erfolgt und welcher nachstehend beschrieben wird. Bei den zusammenwirkenden Komponenten und Elementen handelt es sich um einen feststehenden Hebel, der als Körperelement ausgebildet ist. An das Körperelement sind eine Koppeleinrichtung, eine Verstelleinrichtung, an die sich ein Zug- und Druckelement anschließt und eine Arretierungseinrichtung angeordnet. Dieses Körperelement mit den daran angeordneten Elementen bildet die Basis der Gefäßklemme und der weiteren erfinderischen Tätigkeit. Die erfinderische Entwicklung besteht darin, ein Körperelement mit parallelogrammartigen Gelenkverbindungen zu versehen und eine entsprechende Verstellung für die parallelogrammartigen Gelenkverbindungen auszubilden, um ein parallel fassendes und stufenlos verstellbares Arbeitsmittel zu erhalten. Die Aufgaben bestehen also darin, eine Anordnung der parallelogrammartigen Gelenkverbindungen an dem, zuvor mit verschiedenen Elementen ausgestatteten Körperelement zu finden und eine entsprechende Verstellung dieser Gelenkverbindungen vorzusehen. Diese Aufgaben werden dadurch gelöst, dass das Körperelement der Gefäßklemme Bestandteil des erfinderischen Hebelgetriebes ist.

Erfindungsgemäß werden diese Probleme durch die kennzeichnenden Merkmale des Patentanspruchs 1 gelöst. Vorteilhafte Ausgestaltungen und Weiterbildungen der Erfindung ergeben sich aus den nachfolgenden Unteransprüchen und der Beschreibung.

Die Gefäßklemme umfasst ein Hebelgetriebe, welches aus einem feststehenden Hebel besteht, der als Körperelement ausgebildet ist. Das Körperelement wiederum weist zwei axial verschiebbare Elemente auf, die längs der Mittellinie des Körperelementes, sowie oberhalb und unterhalb des Körperelementes und durch dieses parallel beabstandet, angeordnet sind. Diese beiden beabstandeten Elemente bilden ein Zug- und Druckelement und eine Schub-Zug-Stange, die über ein Verbindungselement fest miteinander verbunden sind. Wird das Zug- und Druckelement über die, am Körperelement angeordnete Verstelleinrichtung verschoben, verschiebt sich gleichzeitig die Schub-Zug-Stange, aufgrund der festen Verbindung zum Zug- und Druckelement, automatisch um den gleichen Betrag. Die Verschiebung des Zug- und Druckelementes kann sowohl vorwärts wie rückwärts erfolgen, wobei die Schub-Zug-Stange ebenfalls dieser Verschiebung folgt. Des Weiteren umfasst das Hebelgetriebe sechs drehbewegliche Hebel, die aus zwei parallel beabstandeten Branchen I, II, zwei Schwenkarmen III, IV und zwei Kniehebeln V, VI gebildet sind. Die Branchen I, II sind längs und parallel zur Mittellinie des Körperelementes beabstandet und über die Schwenkarme III, IV und die Kniehebel V, VI mit dem Körperelement drehbeweglich am freien Ende des Körperelementes verbunden. Die Verbindung der Schwenkarme III, IV und der Kniehebel V, VI am Körperelement verläuft senkrecht zu der am proximalen Ende angeordneten Koppeleinrichtung. Der Schwenkarm III besteht aus einem Paar von Schwenkarmen IIIa, IIIb, die am Körperelement mit der gleichen Gelenkachse V wie der gegenüberliegende Schwenkarm IV verbunden sind. Jeweils eine Branche I, II, ein Schwenkarm III, IV und ein Kniehebel V, VI bilden mit dem Körperelement zusammen eine parallelogrammartige Gelenkverbindung, so dass das Hebelgetriebe aus zwei parallelogrammartigen Gelenkverbindungen besteht.

Zur Befestigung der Schwenkarme III, IV und der Kniehebel V, VI am Körperelement weist dieses drei Gelenkachsen I, I', V und drei Nuten I, II, III auf. Insgesamt sind aber vier Nuten I, II, III, IV im Körperelement ausgebildet. Die erste Nut I ist am distalen Ende des Körperelementes angeordnet und bildet einen Gabelkopf F. Dieser Gabelkopf F dient der Aufnahme eines Schwenkarmes IV, der die Verbindung zwischen dem Körperelement und einer Branche II herstellt. Senkrecht zum Gabelkopf F und der ersten Nut I verläuft die vordere Gelenkachse V, die auch das Paar von Schwenkarmen IIIa, IIIb, welche die Verbindung zwischen dem Körperelement und der anderen Branche I herstellt, aufnimmt. Das Paar von Schwenkarmen IIIa, IIIb ist aber außerhalb bzw. außen am Gabelkopf F auf der vorderen Gelenkachse V drehbeweglich befestigt. Die vordere Gelenkverbindung hat daher am Körperelement die gemeinsame Gelenkachse V. Im Anschluss an die, am distalen Ende des Körperelements angeordnete Nut I bzw. angeordneten Gabelkopf F, folgen in einem bestimmten Abstand zur Gelenkachse V zwei weitere, im Körperelement ausgebildete Nuten II, III. Die zweite Nut II ist oberhalb und die dritte Nut III ist unterhalb des Körperelementes in dieses eingelassen. Die beiden sich gegenüber stehenden und parallel beabstandeten Nuten II, III (zweite und dritte Nut) sind senkrecht zur Mittelinie des Körperelementes angeordnet und weisen jeweils eine Gelenkachse I, I' auf. Diese beiden Gelenkachsen I, I' dienen der drehbeweglichen Befestigung für jeweils einen kurzen Hebelarm Vlla, VIIb eines Kniehebels V, VI. Diese Kniehebel V, VI bilden die Verbindung zwischen dem Körperelement und einer Branche I, II. Die Anordnung der vierten Nut IV wiederum ist vom distalen Ende des Körperelementes und den dazwischen liegenden ; Gelenkachsen I, I' her betrachtet, von diesen beabstandet. Die vierte Nut V verläuft ebenfalls, wie die beiden Nuten II, III für die Gelenkachsen I, I' der Kniehebel V, VI in senkrechter Richtung zur Mittelinie und senkrecht zu den drei, im Körperelement, angeordneten Gelenkachsen I, I', V. Die vierte Nut IV ist durchgängig durch das Körperelement verlaufend, angeordnet, d.h., die Nut IV verbindet die Oberseite und die Unterseite des Körperelementes mit einem durchgehenden Schlitz. Des Weiteren weist der Schlitz eine Schlitzlänge auf, die mindestens der Weglänge s der linearen Verschiebung des Zug- und Druckelementes entspricht, wobei der Schlitz der Führung eines Verbindungselementes dient. Das Verbindungselement sichert die feste Verbindung zwischen dem Zug- und Druckelement und der Schub-Zug-Stange. Eine Verstellung des Zug- und Druckelementes bewirkt zwangsläufig eine Verstellung der Schub-Zug-Stange. Dieses ist auch notwendig, damit die beiden Branchen I, II des Arbeitsmittels sich gleichzeitig aufeinander zu oder voneinander weg bewegen können.

Das Zug- Druckelement ist daher zur Verstellung der ersten Branche I über eine drehbegliche Gelenkachse II, am kurzen Hebelarm Vlla des ersten Kniehebels V, der die Verbindung zur ersten Branche I herstellt, angeordnet. Die mit dem Zug- und Druckelement fest verbundene Schub-Zug-Stange hingegen ist, über eine Gelenkachse II', am kurzen Hebelarm Vllb des zweiten Kniehebels VI, der die Verbindung zur zweiten Branche II herstellt, angeordnet. Ein Kniehebel V, VI ist jeweils aus einem kurzen Vlla, Vllb und einem langen Hebelarm VIIIc, Vllld gebildet, wobei jeweils am langen Hebelarm VIIIc, VIIId ein Gelenkkopf A, A' ausgebildet ist. Ein Gelenkkopf A, A' am freien Ende eines langen Hebelarmes VIIIc, Vllld wird von einem, am proximalen Ende einer Branche I, II angeordneten Gabelkopf B, B', aufgenommen. Ein Gelenkkopf A, A' und ein Gabelkopf B, B' bilden ein Kniegelenk. Die Verbindung des Kniegelenks erfolgt über eine Gelenkachse III, III'. Damit ist eine durchgängige Verbindung, vom Betätigungswerkzeug bis zur Verstellung der Branchen I, II eines Arbeitsmittels gegeben. Jetzt ist noch die parallele Führung der beiden Branchen I, II sicherzustellen. Hierzu sind am Körperelement der Schwenkarm IV und das Paar von Schwenkarmen IIIa, IIIb angeordnet, welche die Verbindung zu den Branchen I, II herstellen. Durch die Anordnung der Schwenkarme III, IV werden die Branchen I, II zwangsgeführt, während der Antrieb für die Verstellung der Branchen I, II aber über die beiden Kniehebel V, VI erfolgt.

Bei den zusammenwirkenden Komponenten und Elementen handelt es sich um den feststehenden Hebel, der als Körperelement ausgebildet ist und um mehrere bewegliche Hebel, bestehend aus dem Zug- und Druckelement, den zwei Kniehebeln V, VI, den zwei Schwenkarmen III, IV und den zwei beweglichen Branchen I, II. Erst die richtige geometrische Auslegung und das richtige vorteilhafte Zusammenspiel dieser Komponenten ermöglichen zwei, sich parallel öffnende und schließende, Branchen I, II an der Gefäßklemme. Auf vorteilhafte Weise bildet das Körperelement bei diesem Hebelgetriebe den feststehenden Hebel und das daran angeordnete Zug- und Druckelement den ersten verschiebbaren Hebel. Dieser erste verschiebbare Hebel ist über einen externen Antrieb gemäß dem Schraub-/Mutterprinzip (Betätigungswerkzeug mit Instrumentengriff) linear verschiebbar. Die Verschiebung des Zug- und Druckelementes wirkt wiederum auf den ersten, um einen Winkel schwenkbaren Kniehebel V der einerseits an dem feststehenden Hebel, gebildet aus dem Körperelement, befestigt ist und andererseits einen, am ersten der beiden Kniegelenke verstellbar angelenkten Hebel trägt, der aus der beweglichen Branche I besteht. Die bewegliche Branche I wirkt wiederum auf die zwei an ihr befestigte Schwenkarme IIIa, IIIb, die als Paar ausgebildet und die ebenfalls am feststehenden Hebel, dem Körperelement, befestigt sind, auf vorteilhafte Weise ein. Da das Zug- und Druckelement fest mit der Schub-Zug-Stange verbunden ist, wirkt die Schub-Zug-Stange, analog zum Zug- und Druckelement, auf den zweiten, um einen Winkel schwenkbaren Kniehebel VI, der einerseits auch am Körperelement befestigt ist und andererseits die zweite der beiden Kniegelenke beweglich angeordnete Branche II, trägt. Die bewegliche Branche II ist über den Schwenkarm IV mit dem Körperelement verbunden.

Zur Funktion des Hebelgetriebes verfügt dieses somit über sechs bewegliche Hebel. Aufgrund der symmetrischen Eigenschaften des Hebelgetriebes sind jeweils drei Hebel auf der einen Seite der Mittelinie des Körperelementes und drei Hebel spiegelsymmetrisch auf der anderen Seite der Mittelinie des Körperelementes angeordnet. Drei der auf einer Seite (oberhalb) der Mittelinie drehbeweglich angeordneten Hebel, sind untereinander mit fünf Gelenkachsen I, II, III, IV, V verbunden. Von diesen fünf Gelenkachsen I, II, III, IV, V sind zwei Gelenkachsen I, V im feststehenden Körperelement angeordnet und dienen dem daran befestigten Paar von Schwenkarmen IIIa, IIIb und dem ersten Kniehebel V als Drehpunkte. Die anderen drei Gelenkachsen II, III, IV sind beweglich angeordnet und können um einen Winkel innerhalb eines bestimmten Winkelbereiches verschoben werden. Gleiches gilt für die, auf der anderen Seite der Mittelinie angeordneten drei Hebel, d.h., die sich an der Mittellinie symmetrisch gegenüberliegenden Hebel sind deckungsgleich.

D.h., dass das feststehende Körperelement mit dem ersten, daran angeordneten Schwenkarm III und dem ersten, daran angeordneten Kniehebel V über die fünf Gelenkachsen I, II, III, IV, V mit der ersten Branche I verbunden ist und mit dem zweiten, daran angeordneten Schwenkarm IV und dem zweiten Kniehebel VI über die fünf Gelenkachsen I', II', III', IV', V mit der zweiten Branche II verbunden ist, wobei drei Gelenkachsen I, I', V, im Körperelement feststehend, als Drehpunkt für die zwei Schwenkarme III, IV und als Drehpunkt für die zwei Kniehebel V, VI dienen und sechs Gelenkachsen II, II', III, III', IV, IV' beweglich auf jeweils einem Kreisbogen angeordnet, um die Winkel α, β, γ, α', β', γ' verschiebbar sind.

Dieses Hebelgetriebe wandelt vorteilhafterweise eine lineare Bewegung bzw. eine Verschiebung des Zug- und Druckelementes und der Schub-Zug-Stange in eine resultierende Verschiebung der zwei beweglichen Branchen I, II um. Die lineare Bewegung des Zug- und Druckelementes erfolgt prinzipiell durch einen Schraub-/Mutterantrieb. Der Antrieb des Schraub-/Mutterantriebes wird durch eine Drehbewegung bzw. durch eine bestimmte Anzahl von Umdrehungen des Instrumentengriffes am Betätigungswerkzeug erzeugt, wobei die Umdrehungen des Betätigungswerkzeuges auf die Verstelleinrichtung übertragen wird. Die Drehbewegungen des Betätigungswerkzeuges können sowohl vorwärts wie rückwärts bzw. links- oder rechtsherum erfolgen. Eine Rechtsdrehung am Betätigungswerkzeug lässt das Zug- und Druckelement und die Schub-Zug-Stange um eine bestimmte Weglänge s zurückziehen. Beim Zurückziehen des Zug- und Druckelementes und der Schub-Zug-Stange öffnen sich die Branchen I, II des Arbeitsmittels. Eine Linksdrehung am Betätigungswerkzeug erzeugt eine lineare Verschiebung des Zug- und Druckelementes, sowie der Schub-Zug-Stange, um eine bestimmte Weglänge s in Vorwärtsrichtung. Beim Vorwärtsschieben des Zug- und Druckelementes und der Schub-Zug-Stange schließen sich die Branchen I, II des Arbeitsmittels. Durch eine Drehbewegung am Betätigungswerkzeug wird eine resultierende Verschiebung der Branchen I, II in zwei Richtungen, eine Schließrichtung oder eine Öffnungsrichtung, bewirkt. Das Verhältnis der Drehbewegungen am Betätigungswerkzeug zu der linearen Verschiebung des Zug- und Druckelementes und somit auch zu der Schub-Zug-Stange ist ca. 1:0,4. Hierbei handelt es sich um ein Untersetzungsverhältnis bei dem Schraub-/Mutterantrieb. Diese Untersetzung ist Voraussetzung für eine gute Einstellung der Kraftübertragung auf die Gefäßklemme. Eine Vielzahl von Umdrehungen am Betätigungswerkzeug erzeugt eine geringe lineare Verschiebung des Zug- und Druckelementes sowie der Schub-Zug-Stange. Das erfindungsgemäße Hebelgetriebe setzt diese relativ geringe lineare Verschiebung (Weglänge s) des Zug- und Druckelementes, aufgrund eines Übersetzungsverhältnisses des Hebelgetriebes von ca. 1:12, wieder in eine größere Öffnung des Arbeitsmittels (Weglänge X) bzw. Verschiebung der Branchen I, II, um. Für diese Hebelübersetzung ist eine erfinderische Formgestaltung der Kniehebel V, VI erforderlich. Ein Kniehebel V, VI besteht daher aus einem abgeknickten Gelenkhebel. Ein solcher abgeknickter Gelenkhebel wird aus einem kurzen Vlla, Vllb und einem langen Hebelarm VIIIc, VIIId gebildet. Der lange Hebelarm VIIIc, Vllld eines Kniehebels V, VI weist am freien Ende einen Gelenkkopf A, A' mit Auge auf, während am kurzen Hebelarm Vlla, Vllb eine Feder I, II mit zwei Öffnungen, zur Aufnahme von zwei Gelenkachsen I, II, I', II' angeordnet ist. Die zwei Schwenkarme III, IV und die zwei Kniehebel V, VI sind drehbeweglich über die Gelenkachsen III, III', IV, IV' Träger der verschiebbaren Branchen I, II. Die verschiebbaren Branchen I, II weisen an einem Ende einen Gabelkopf B, B' mit einer senkrecht dazu angeordneten Öffnung, zur Aufnahme einer Gelenkachse III, III' für ein Kniegelenk, und, dazu beabstandet, eine Öffnung zur Aufnahme einer Gelenkachse IV, IV' für einen Schwenkarm III, IV auf.

Eine lineare Verschiebung des Zug- und Druckelementes und der Schub-Zug-Stange auf einer Weglänge s, hat eine Winkelverschiebung um die Gelenkachsen I, I', der Kniehebel V, VI im Winkelbereich α, β, α', β' und eine Winkelverschiebung der zwei Gelenkachsen IV, IV' der Schwenkarme III, IV mit den beweglichen Branchen I, II im Winkelbereich γ, γ' zur Folge.

Am Beispiel eines geöffneten Arbeitsmittels soll beschrieben werden, welchen Weg die beiden parallel beabstandeten und verschiebbaren Branchen I, II aus der geöffneten Stellung bis zur geschlossen Stellung zurücklegen. Die beiden verschiebbaren Branchen I, II bilden zwei Geraden, die in die gleiche Richtung weisen, wobei die Geraden nicht nebeneinander, sondern übereinander parallel beabstandet sind, d.h., dass jede Branche I, II in einer eigenen Ebene liegt, wobei die Ebenen zueinander parallel angeordnet sind. In der oberen Ebene befindet sich die obere verschiebbare Branche I und in der unteren Ebene die untere verschiebbare Branche II. Die Branchen I, II stehen sich, in der geöffneten Stellung des Arbeitsmittels, annähernd parallel beabstandet gegenüber. Wird nun das Arbeitsmittel, wie zuvor beschrieben, geschlossen, verschieben sich, aufgrund der Hebelwirkung der Schwenkarme III, IV und Kniehebel V, VI, die Branchen I, II. Die Branchen I, II verschieben sich jeweils in ihrer Ebene nach vorne und gleichzeitig erfolgt eine Verschiebung der Branchen I, II und somit der Ebenen, aufeinander zu. Daher versteht man unter der Schließbewegung des Arbeitsmittels eine Parallelverschiebung der beweglichen Branchen I, II in einer Ebene, z.B. in horizontaler X-Richtung und eine Verschiebung der Ebenen aufeinander zu. Darunter wird eine Parallelverschiebung zweier Ebenen aufeinander zu, z.B. in senkrechter Z-Richtung, verstanden. Eine horizontale Verschiebung der beweglichen Branchen I, II in Y-Richtung findet nicht statt. Da die beiden Bewegungen der verschiebbaren Branchen I, II in X- und Z- Richtung gleichzeitig erfolgen, ergibt sich für die Verschiebung der beweglichen Branchen I, II eine resultierende Richtung, die sich aus den Anteilen der X- und Z- Richtung zusammensetzt. D.h., die obere Ebene, gebildet aus der Fläche (Xₒ mal Yₒ), nähert sich der unteren, parallel beabstandeten Ebene, gebildet aus der Fläche (Xᵤ mal Yᵤ) über eine dazwischen liegende senkrechte Ebene, gebildet aus der Fläche (Xₙ mal Zₙ), an. Die Verschiebung der oberen Ebene in Richtung (X, Z) der unteren Ebene, erfolgt auf der resultierenden Richtung Δᵣₑₛ= ΔX + ΔZ, die in der aufgespannten Ebene (Xₙ mal Zₙ) liegt. In diese resultierende Richtung Δᵣₑₛ= ΔX + ΔZ verschieben sich die beweglichen Branchen I, II, aufgrund der Bewegung der Kniehebel V, VI], aufeinander zu oder entfernen sich von dieser reziprok.

In geöffneter Stellung des Arbeitsmittels bildet die verlängerte Mittellinie des Körperelementes mit den, über die Schwenkarme III, IV angelenkten beweglichen Branchen I, II, die beide parallel beabstandet sind, im Prinzip zwei virtuelle Trapeze. Da die beiden Trapeze spiegelsymmetrisch sind, muss als Beispiel nur ein Trapez beschrieben werden. Anders dargestellt bildet die Mittellinie des Körperelementes mit ihren Funktionsbereichen I und II, (zu den Funktionsbereichen nachstehend mehr), die Grundlinie a eines virtuellen Trapezes. Die Grundlinie a mit den Eckpunkten A und B weist im Eckpunkt B die Gelenkachse V auf, welche den unteren Drehpunkt der Schwenkarme III, IV bildet. Die Schwenkarme III, IV selbst bilden die rechte Seitenlinie c bzw. c' der Trapeze, die durch die Eckpunkte B und C bzw. C' begrenzt wird. Die Seitenlinie c weist im Eckpunkt C die Gelenkachse IV mit einem oberen Drehpunkt für den Schwenkarm III auf. Zur Grundlinie a ist die Decklinie b bzw. b', welche durch die bewegliche Branche I, II gebildet wird, parallel beabstandet. Die Decklinie b mit den Eckpunkten C und D, weist im Eckpunkt C die Gelenkachse IV auf, die den oberen Drehpunkt des Schwenkarms III bildet, während sich am freien Ende der bewegtichen Branche I der Eckpunkt D befindet. Zwischen den Eckpunkten D und A befindet sich die gedachte linke Seitenlinie d (Öffnung des Arbeitsmittels) des Trapezes. Der Eckpunkt D steht nicht senkrecht über dem Eckpunkt A, sowie ebenfalls nicht der Eckpunkt C über dem Eckpunkt B, wobei die Eckpunkte B, C und C' die gelenkige Verbindung zwischen den Branchen I, II des Arbeitsmittels bilden. Beim Schließen des Arbeitsmittels ist der Bewegungsablauf, bezogen auf die Betrachtung eines Trapezes, folgender: Beim Schwenken des Kniehebels V, VI in Vorwärtsrichtung, Winkel α, α' und Winkel β, β' verkleinern sich, verschiebt dieser die bewegliche Branche I, II um den Drehpunkt in der Gelenkachse III III', wodurch eine Verschiebung der gedachten Decklinie b bzw. b' erfolgt. Aufgrund der Tatsache, dass die bewegliche Branche I, II einen weiteren Drehpunkt C, C' in der Gelenkachse IV, IV' aufweist, der zum Drehpunkt in Gelenklachse III, III' mit einem bestimmten Maß beabstandet ist, wird die bewegliche Branche I, II zwangsgeführt. Die durch die beiden Gelenkachsen III, III' und IV, IV' am Kniehebel V, VI und den Schwenkarmen III, IV befestigte und beweglich geführte Branche I, II führt, aufgrund der Zweipunktführung, eine gleichbleibende Bewegung aus. Die Branche I, II folgt dieser Bewegung, die durch den Schwenkarm III, IV und den Kniehebel V, VI vorgegeben wird. Da der Schwenkarm III, IV auf der Gelenkachse V und der Kniehebel V, VI auf der Gelenkachse I, I' des feststehenden Hebels bzw. des Körperelementes drehbeweglich befestigt sind, kann sich die bewegliche Branche I, II nur auf einem Kreisbogen geführt bewegen. Diese Bewegung führt die bewegliche Branche I parallel auf die gegenüberliegende bewegliche Branche II zu oder parallel von dieser weg. Die Größe des Kreisbogens wird durch die vorgegebene Länge des Schwenkarms III, IV und des Kniehebels V, VI bestimmt. Bezogen auf die bildliche Darstellung mit dem Trapez bedeutet das, dass sich die verschiebbaren Branchen I, II beim Schließen des Arbeitsmittels immer parallel zueinander nähern oder entfernen, je nachdem, ob das Arbeitsmittel geöffnet oder geschlossen wird. D.h., die Grundlinie a liegt in einer Ebene und die Decklinie b, b' liegt in jeweils einer Ebene, wobei die drei Ebenen parallel zueinander beanstandet sind. Die in der Grundebene (Grundlinie a) liegende Mittellinie des Körperelementes und die zwei, in der Deckebene (Decklinie b, b') liegenden verschiebbaren Branchen I, II, können sich parallel annähern oder entfernen. Beide Branchen I, II weisen, in ihren Ebenen liegend, die gleiche Richtung (X-Richtung) auf. In Längsrichtung (X-Richtung), zum freien Ende der beiden Branchen I, II hin betrachtet, sind bei geöffneter Stellung des Arbeitsmittels und größtmöglichem Abstand der verschiebbaren Branchen I, II zu der Mittellinie des Körperelementes, also dem größtmöglichen Abstand zwischen den beiden Ebenen, die freien Enden der beiden Branchen I, II bzw. die beiden Eckpunkte A und D, bzw. D' am weitesten voneinander entfernt und stehen nicht senkrecht übereinander, obwohl die freien Enden der beiden Branchen I, II und somit deren Eckpunkte D, D' immer senkrecht zueinander stehen. Erst beim Schließen des Arbeitsmittels bzw. beim Verschieben der beweglichen Branchen I, II und somit der Deckebenen (Decklinie b, b') auf die Grundebene (Grundlinie a) zu, nähert sich der Eckpunkt D, D' dem Eckpunkt A, wie zuvor beschrieben, über eine Kreisbahn an. D.h., beim Schließen des Arbeitsmittels verschieben sich die beweglichen Branchen I, II einerseits in ihrer Deckebene (Decklinie b, b'), und andererseits nähern sich die Deckebenen (Decklinie b, b') der Grundebene (Grundlinie a) parallel an. Liegen die Ebenen b, b' an der Ebene a an, sind die Branchen I, II und somit das Arbeitsmittel geschlossen. Gleiches trifft im umgekehrten Sinne zu, wenn das Arbeitsmittel geöffnet wird und sich die verschiebbaren Branchen I, II voneinander wegbewegen. Die Deckebene (Decklinie b, b') verschiebt sich dann parallel zur Grundebene (Grundlinie a) auf einem Radius, der auf einem Kreisbogen bis zur maximalen Endstellung geführt wird. Im Grunde liegt bei der Erfindung eine Verschiebung zweier beweglicher Branchen I, II in einer Ebene und eine Verschiebung dieser Ebenen auf eine mittlere Ebene zu, oder von dieser weg, vor. Aufgrund dieser gleichzeitigen Verschiebung der Branchen I, II in zwei unterschiedliche Richtungen, entsteht eine resultierende Verschiebung der beweglichen Branchen I, II auf einem Kreisbahnausschnitt. Diese alternierende Bewegung der verschiebbaren Branchen I, II zum Öffnen und Schließen einer Gefäßklemme wird durch das erfindungsgemäße Hebelgetriebe erzeugt. Aufgrund der erfinderischen Anordnung und Auslegung der Hebelmechanik für die öffnende und schließende Bewegung der verschiebbaren Branche im Arbeitsmittel, erfolgt die Bewegung gleichzeitig in horizontaler und vertikaler Richtung.

Bei dem Übergang von einer geschlossenen Stellung des Arbeitsmittels zu einer geöffneten Stellung betrifft es die umgekehrte Bewegungsrichtung. Die beweglichen Branchen I, II werden auf der, aus der X- und Z-Richtung gebildeten resultierenden Richtung, parallel zu der Mittellinie des Körperelementes geführt, wobei sich gleichzeitig die beiden freien Enden der beweglichen Branchen I, II vom Eckpunkt A des Trapezes entfernen. Diese Verschiebung der beweglichen Branchen I, II von der Mittellinie des Körperelementes weg erfolgt nicht senkrecht, sondern, wie zuvor aufgezeigt, auf einem resultierenden Kreisbogen.

Die lineare Bewegung des Zug- und Druckelementes ist in jeder Stellung alternierend, und somit auch die Bewegung der Schub-Zug-Stange, wodurch ein Öffnen und Schließen des Arbeitsmittels der Gefäßklemme in jeder Stellung zwischen maximaler Öffnung und geschlossenen Branchen I, II, erfolgen kann. Die Kniehebel V, VI werden dazu, ähnlich einem Kurbeltrieb, vom Zug- und Druckelement sowie von der Schub-Zug-Stange, um einen Winkel ϑ, ϑ' in einem bestimmten Winkelbereich α, α' verschoben. Dabei wird der lineare Weg s des Zug- und Druckelementes und der Schub-Zug-Stange in einen kreisförmigen Weg b geändert, wobei die Länge b der Bogenlänge auf einem Kreisbogen entspricht. Die Länge b korrespondiert annähernd mit der Länge s und bestimmt somit den möglichen verstellbaren Winkelbereich α, α' der Kniehebel V, VI, wobei b auch proportional zum Winkel α, α' und zum Radius r ist. Der Radius r wird bestimmt durch den Abstand der beiden Gelenkachsen I, II und I', II', die an den Kniehebeln V, VI angeordnet sind. Die Gelenkachse I, I' bildet jeweils den Drehpunkt des Kniehebels V, VI und ermöglicht eine schwenkbare Bewegung desselben um diesen Drehpunkt, wobei die Gelenkachse I, I' fest im Körperelement angeordnet ist. Die Gelenkachse II, II' bildet zu der Gelenkachse I, I' an den Kniehebeln V, VI einen beabstandeten Angriffspunkt für das Zug- und Druckelement sowie für die Schub-Zug-Stange. Das Zug- und Druckelement ist also an der Gelenkachse II mit dem Kniehebel V verbunden und kann den Kniehebel V mit Hilfe der Gelenkachse II vorwärts und rückwärts verschieben. Da die Schub-Zug-Stange an der Gelenkachse II' mit dem Kniehebel VI verbunden ist, kann diese den Kniehebel VI mit Hilfe der Gelenkachse II' vorwärts und rückwärts verschieben. Die Gelenkachsen II, II' sind aufgrund der Verschiebung entlang eines Kreisbogens beweglich bzw. verschiebbar angeordnet. Die Größe der Verschiebung der Kniehebel V, VI wird durch den Abstand der beiden Gelenkachsen I, II und I', II' und den linearen Weg s des Zug- und Druckelementes bestimmt. D.h., aus den beiden Parametern r und s ergibt sich ein bestimmter Winkel α, α' der den Schwenkbereich der Kniehebel V, VI und somit den Schwenkbereich der Gelenkachse II, II' und III, III' bestimmt. Der Schwenkbereich ergibt sich also aus der Länge des linearen Weges s des Zug- und Druckelementes, der ca. 5 mm beträgt und dem Abstand r der beiden Gelenkachsen I, II und I', II', der ebenfalls ca. 5 mm beträgt. Der sich daraus ergebende Schwenkbereich für die Kniehebel V, VI weist einen Winkelbereich α, α' zur Verstellung der Kniehebel V, VI und somit zur Verstellung der, an den Kniehebeln V, VI angeordneten Branchen I, II, von ca. 0 Grad bis zu 90 Grad, vorzugsweise von 10 Grad bis zu ca. 80 Grad, auf. Des Weiteren weisen die Kniehebel V, VI jeweils eine Gelenkachse III, III' auf, um welche jeweils eine Branche I, II wiederum beweglich angeordnet ist. Aufgrund einer vorgegebenen Länge L eines Kniehebels V, VI und der vorgegebenen Länge der Schwenkarme III, IV kann das Arbeitsmittel der Gefäßklemme eine variable Öffnung zwischen den beweglichen Branchen I, II im Bereich von 0 mm bis ungefähr 60 mm einnehmen. Jede Änderung der Öffnungsgröße zwischen 0 mm und 60 mm zwischen den beiden Branchen I, II kann stufenlos über das verstellbare Zug- und Druckelement erfolgen. Eine zusätzliche Änderung der Öffnungsgröße kann über die konstruktive Änderung eines Parameters, dem Achsabstand r zwischen der Gelenkachse I, I' und der Gelenkachse II, II' erreicht werden, wodurch sich der Weg des Bogenmaßes b der Gelenkachse II, II' aufgrund eines größeren Radius r auf dem Umfang des Kreisbogens, verändern kann. Eine weitere konstruktive Änderung der Öffnungsgröße kann sich auch durch eine Änderung der linearen Weglänge s des Zug- und Druckelementes ergeben, die eine Änderung der Bogenlänge b ermöglicht. Des Weiteren können die Längen der Hebelarme (Schwenkarme III, IV und Kniehebel V, VI) konstruktiv verändert werden. Auch die Veränderung aller drei Parameter ist möglich und führt zu einer Änderung der Größe der Öffnung des Arbeitsmittels und somit der Gefäßklemme. In der Regel ist eine Öffnung der Gefäßklemme im Bereich von 0 mm bis zu 40 mm zum Erfassen von Gewebe u.ä für den Chirurgen ausreichend. Bei Bedarf können Gefäßklemmen mit anderen Öffnungsgrößen am Arbeitsmittel eingesetzt werden, die, aufgrund der Kompatibilität mit den vorhandenen Instrumenten, nur ausgetauscht werden müssen.

Das Körperelement als feststehender Hebel und Teil des Hebelgetriebes einer Gefäßklemme, bildet dabei die Basis des Instrumentes und ist Träger aller Komponenten bzw. aller Elemente, die mit dem Körperelement fest oder beweglich verbunden sind. Eine Ausnahme bilden die beweglich angeordneten Branchen I, II, die indirekt über die Hebelmechanik mit dem Körperelement in Beziehung stehen bzw. verbunden sind.

Das Körperelement ist vorteilhafterweise aus einem einstückigen, festen korrosionsarmen Metall, vorzugsweise aus Edelstahl, Titan oder Tantal hergestellt. Es weist zwei Enden auf, die sich auf der gedachten geraden Mittellinie befinden. Des Weiteren ist das einstückige Körperelement relativ länglich und schmal ausgebildet. Dies bedeutet, auf die Größe des Querschnittes bezogen, Maße im Bereich von ca. 5 mm x ca. 9 mm, wobei 9 mm die Höhe und die 5 mm die Breite des Körperelementes bilden, ohne Berücksichtigung der Verstelleinrichtung und Koppeleinrichtung. Bezogen auf die länglichen Maße ergibt sich die Größe in einem Bereich von ca. 50 mm bis 60 mm, vorzugsweise im Bereich um die 55 mm. Die Gefäßklemme weist im Prinzip drei Funktionsbereiche I, II, III auf. Die drei Funktionsbereiche I, II, III betreffen das Arbeitsmittel im Funktionsbereich I, das Hebelgetriebe im Funktionsbereich II und einen Trägerteil im Funktionsbereich III. Der erste Funktionsbereich I, der das Arbeitsmittel der Gefäßklemme betrifft, weist die zwei parallel verschiebbaren Branchen I, II auf. Die beiden Branchen I, II weisen eine Form auf, die vom freien Ende des Körperelementes zum freien distalen Ende des Arbeitsmittels hin geradlinig und verjüngend verläuft. Des Weiteren sind die im Funktionsbereich I angeordneten, verschiebbaren Branchen I, II mit den Schwenkarmen III, IV beweglich verbunden. Stirnseitig ist das frei stehende distale Ende des Arbeitsmittels abgerundet, um Verletzungen z.B. am Gewebe zu vermeiden. Beide Branchen I, II sind mit einer vorteilhaften, aus dem Stand der Technik bekannten Verzahnung und/oder mit einem auswechselbaren Polster versehen, wobei die Länge der Branche I, II ca. ¼ bis ½, vorzugsweise ca. 1/3 der Gesamtlänge der Gefäßklemme beträgt. In einer anderen Ausführungsform kann die Länge der Verzahnung die Hälfte der Gesamtlänge der Gefäßklemme ausmachen. Die Breite der Branche I, II, die sich zum freien Ende hin verjüngt, ist auf ihrer Länge schmaler als die Breite des Mittelteiles und die Breite des Trägerteiles und somit der schmalste Bereich der Gefäßklemme. Der zweite Funktionsbereich II besteht aus dem Mittelteil. Der Mittelteil zeigt größtenteils die erfindungsgemäße Hebelmechanik auf. Die Hebelmechanik im Mittelteil der Gefäßklemme besteht aus den zwei angelenkten Schwenkarmen III, IV und den zwei angelenkten Kniehebeln V, VI, wobei die Schwenkarme III, IV und die Kniehebel V, VI einerseits am Körperelement und andererseits an den Branchen I, II angelenkt sind. Ein Schwenkarm III, IV und ein Kniehebel V, VI sind annähernd parallel beabstandet und weisen somit in die gleiche Richtung. Jeder dieser Schwenkarme III, IV und Kniehebel V, VI ist an dem Körperelement beweglich angeordnet und erstreckt sich in Richtung der, parallel zum Körperelement angeordneten Branchen I, II. Der Mittelteil der Gefäßklemme weist an der entsprechenden Stelle am Körperelement die Gelenkachse V auf, an welcher die beiden Schwenkarme III, IV drehbeweglich angeordnet sind. Diese bewegliche Anordnung bzw. gelenkige Verbindung ermöglicht es den Schwenkarmen III, IV, eine rotative Bewegung um den Drehpunkt der Gelenkachse V auszuführen. Die rotative Bewegung der Schwenkarme III, IV ist aber auf eine Schwenkbewegung zwischen ca. 0 Grad und 90 Grad begrenzt. Aufgrund der alternierenden linearen Bewegung des Zug- und Druckelementes, ist eine Schwenkbewegung der Schwenkarme III, IV in beide Richtungen, vorwärts und rückwärts, also mit und gegen den Uhrzeigersinn, möglich. Für die Bewegungsrichtung existiert nur ein Freiheitsgrad, weitere Freiheitsgrade bestehen nicht. Aufgrund der Schwenkbewegung der Schwenkarme III, IV ist ein paralleles Öffnen und Schließen des Arbeitsmittels durch die beweglich angeordnete Branche I, II möglich. Die Schwenkbewegung der Schwenkarme III, IV ist zwangsgeführt und wird durch die Verschiebung der Kniehebel V, VI erzwungen. Da ein Paar von Schwenkarmen IIIa, IIIb am freien Ende des Körperelementes angelenkt sind, können diese, beim Schließen des Arbeitsmittels bzw. beim Schließen der beweglichen Branchen I, II, den Schwenkarm IV, der mittig am freien Ende des Körperelementes angeordnet ist, zwischen sich aufnehmen. Die Breite des Mittelteiles ist auf seiner Länge schmaler als die Breite des Trägerteiles, aber auch breiter als die Breite der beweglichen Branchen I, II, wobei die Länge des Mittelteiles ca. 1/8 bis 3/8, vorzugsweise ca. 1/3 der Gesamtlänge der Gefäßklemme, beträgt und einen in etwa quadratischen Querschnitt aufweisen kann, weil die Höhe und Breite des Querschnittes des Mittelteils in etwa gleich ist.

Das andere freie Ende der Gefäßklemme zum proximalen Ende hin, welches im Funktionsbereich III liegt, weist einen, in etwa rechteckigen Querschnitt auf. Die Breite des Querschnittes ist kleiner ist, als die Höhe des Querschnitts. Des Weiteren ist der Querschnitt am proximalen Ende der Branche in etwa doppelt so groß wie der Querschnitt am distalen Ende. Die Länge des rechteckigen Querschnitts beträgt ca. ¼ bis ½, vorzugsweise ca. 1/3 der Gesamtlänge der Gefäßklemme, was den Funktionsbereich III darstellt und als Trägerteil ausgebildet ist. Aufgrund des rechteckigen Querschnitts sind vier Seiten mit jeweils einer ebenen Fläche, zwei Seitenflächen, einer Fläche an der Unterseite und einer Fläche an der Oberseite vorhanden, die alle für verschiedene Funktionen benötigt und genutzt werden. Das Trägerteil weist an den zwei sich parallel beabstandeten Seitenflächen, zum proximalen Ende des Körperelementes hin, jeweils eine Haltebacke auf. Die beiden parallel beabstandeten und sich gegenüberliegenden Haltebacken sind einstückig mit dem Körperelement verbunden und bilden die Kupplungsmittel der Koppeleinrichtung. Die Koppeleinrichtung dient der, an der Bedieneinrichtung (Trägerinstrument) angeordneten Greifeinrichtung, zum Fassen und Lösen der Gefäßklemme. Beim Erfassen der Gefäßklemme wird gleichzeitig das Betätigungswerkzeug mit Instrumentengriff zum Verstellelement zentriert und in Eingriff gebracht. Das Verstellelement ist Teil der Verstelleinrichtung, die auf der Oberseite des Trägerteiles im Funktionsbereich III angeordnet ist, wobei die, auf der Oberseite angeordnete Halterung für die Verstelleinrichtung einstückig mit dem Körperelement verbunden ist.

Des Weiteren befindet sich im Funktionsbereich III am proximalen Stirnende eine Arretierungseinrichtung. Die Arretierungseinrichtung wird aus einer Blattfeder und einem, in einer Bohrung geführten Arretierungsstift, gebildet, der in ein, in einem Verstellelement angeordneten, Rastprofil eingreift. Das Rastprofil im Verstellelement besteht im Prinzip aus aneinander gereihten Sackbohrungen, bzw. nur aus den angedeuteten Bohrungen geringer Tiefe. Die angedeuteten Bohrungen werden durch die Zentrierspitze eines Bohrers hergestellt. Die Zentrierspitze eines Bohrers erzeugt im Prinzip eine kegelförmige Vertiefung. Diese Vertiefungen sind auf dem Umfang des Verstellelementes angeordnet. In jeweils eine dieser Vertiefungen greift ein Arretierungsstift ein. Die Blattfeder der Arretierungseinrichtung ist an der Unterseite des Körperelementes im Bereich des Trägerteiles befestigt und übt eine bestimmte Kraft auf den Arretierungsstift aus, so dass dieser immer in eine Vertiefung im Verstellelement eingreift. Stirnseitig ist das frei stehende, proximale Ende des Trägerteiles, in dem sich die Bohrung für den Arretierungsstift befindet, abgerundet, um Verletzungen z.B. am Gewebe zu vermeiden.

Auf der Oberseite des Körperelementes befindet sich im Funktionsbereich III, im Anschluss an das Trägerteil, die Verstelleinrichtung mit dem Zug- und Druckelement. Das Zug- und Druckelement ist einerseits über ein Verbindungselement mit dem Verstellelement der Verstelleinrichtung und andererseits an der, dem Verbindungselement gegenüberliegenden Seite, mit dem Kniehebel V aus der Hebelmechanik verbunden. Bei dem Kniehebel V handelt es sich um einen abgeknickten Hebel, einen sogenannten Winkelhebel. Der Winkelhebel wird aus den zwei Hebelarmen Vlla, VIIIc, gebildet, wobei die Länge der Hebelarme unterschiedlich ist. Das Verhältnis der Länge der Hebelarme zueinander beträgt zwischen dem kurzen und langen Arm ungefähr 1:4. Der Winkel zwischen dem kürzeren VIIa und längeren Hebelarm VIIIc des Kniehebels V beträgt in etwa 100 Grad bis 150 Grad, vorzugsweise 135 Grad. Des Weiteren ist der, aus den zwei Hebelarmen VIIa, VIIIc bestehende Kniehebel V, als Gelenkhebel ausgebildet. Am kürzeren Hebelarm VIIa sind zwei Öffnungen, vorzugsweise ausgebildet als Bohrungen, angeordnet, welche zur Aufnahme jeweils einer der Gelenkachsen I, II dienen. Des Weiteren ist der kürzere Hebelarm VIIa schmaler als der längere Hebelarm VIIIc des Kniehebels V ausgebildet. Der schmalere kurze Hebelarm VIIa weist die Form der Feder I auf, die einerseits in die Nut II, die im Funktionsbereich III des Trägerteiles angeordnet ist und andererseits in eine Nut des gabelartig ausgeformten Zug- und Druckelementes, eingreift. Das andere Ende des Kniehebels V weist einen Gelenkkopf A mit Auge auf. Der Gelenkkopf A bildet mit dem gabelartig ausgeformten Ende der beweglichen Branche I eine gelenkige Verbindung, bzw. ein Scharnier, welches als verbindendes Gelenk zwei Teile (Branche I und Kniehebel V) beweglich miteinander verbindet. Die zuvor zum Zug- und Druckelement gemachten Angaben treffen analog auf die am Körperelement angeordnete Schub-Zug-Stange zu. Die Zug-Schub-Stange ist über ein Verbindungselement fest mit dem Zug- und Druckelement verbunden. Des Weiteren ist die Schub-Zug-Stange mit dem Kniehebel VI aus dem Hebelgetriebe verbunden. Der Kniehebel VI weist die gleichen Abmessungen und Eigenschaften auf, wie der, am Zug- und Druckelement angeordnete Kniehebel V und muss daher nicht noch einmal beschrieben werden. Auch dieser an der Schub-Zug-Stange angeordnete Kniehebel VI weist einen Gelenkkopf A' auf, der mit einer Branche II in Verbindung steht.

Das Gelenk nimmt sowohl die Last des beweglichen Teils und auch die Kraft der Bewegung auf, wobei das Gelenk nur einen Freiheitsgrad aufweist. Die Eingangsgröße für die Kraft wird durch das Betätigungswerkzeug mit Instrumentengriff bestimmt und wirkt über das Schraub-/Muttergetriebe, der linearen Verstellung des Zug- und Druckelementes und der Hebelmechanik auf die parallel verstellbaren Branchen I, II ein. Aufgrund der Untersetzung durch das Schraub-/Muttergetriebe wird die vom Instrumentengriff erzeugte Kraft erst verstärkt und anschließend aufgrund der Übersetzung der Hebelmechanik, wieder etwas verringert, wobei die Hebelmechanik die Ausgangsgröße der Kraft entlang des Weges und somit das veränderliche Verhältnis bestimmt.

Ein Gabelkopf B, B' und ein Gelenkkopf A, A' sind über die Gelenkachse III, III' miteinander gelenkig verbunden, wobei nur die Kniehebel V, VI eine Drehbewegung um den Drehpunkt der Achse III, III' vollziehen. D.h., dass nur die Gelenkköpfe A, A' der Kniehebel V, VI eine Drehbewegung in den Gabelköpfen B, B' der Branchen I, II vornehmen können. Die Begründung dafür ist, dass die beweglichen Branchen I, II über die Schwenkarme III, IV mit dem feststehenden Hebel, des Körperelementes, verbunden sind. Aufgrund dieser Verbindung werden die beweglichen Branchen I, II zwangsgeführt. Die Zwangsführung stellt die parallele Stellung der verschiebbaren Branchen I, II immer sicher. Aufgrund der parallelen Stellung der beweglichen Branchen I, II, egal, ob das Arbeitsmittel ganz geöffnet oder ganz geschlossen ist oder aufeinander zu oder voneinander weg geführt wird, bleiben die Gabelköpfe B, B' in einer bestimmten Stellung und nur die Gelenkköpfe A, A' der Kniegelenke vollziehen eine Drehbewegung. Die Drehbewegung der Kniehebel V, VI ist aber auf einen bestimmten Winkelbereich festgelegt. Dieser Winkelbereich zwischen den beweglichen Branchen I, II und den Kniegelenken beträgt ungefähr zwischen 90 Grad und 180 Grad. Die Gelenkköpfe A, A' an den langen Hebelarmen VIIIc, Vllld der Kniehebel V, VI können in den Gabelköpfen B, B' der Branchen I, II eine Verschwenkung im vorgenannten Winkelbereich von bis zu 90 Grad, ausführen. Bei der Schwenkbewegung der Kniehebel V, VI werden die Schwenkarme III, IV der Branchen I, II und die Kniehebel V, VI, im vorgenannten Winkelbereich, um ca. 90 Grad aufeinander zu oder voneinander weg geschwenkt.

Die erfinderische Gefäßklemme, insbesondere eingesetzt als Aortenklemme bei laparoskopischen Operationen an der infrarenalen Aorta, gewährleistet mit Hilfe einer Bedieneinrichtung und einem Betätigungswerkzeug, ein sicheres Abklemmen auch sklerotischer Gefäße. Ein Drehverschluss mit Feingewinde erlaubt ein dosiertes Schließen und Öffnen der parallel verschiebbaren Branchen I, II einer Gefäßklemme. Und durch den Einsatz von auswechselbaren Polstereinsätzen an den Branchen I, II der erfindungsgemäßen Gefäßklemme wird ein atraumatisches Klemmen von Gefäßen verbessert. Auch die Einstellung der Druckkraft, die über das Hebelgetriebe, aufgrund der Übersetzungen, dosiert auf ein Gewebe oder Gefäß eingestellt werden kann, ermöglicht, bei einem Gefäß den Durchfluss einer Flüssigkeit zu regeln. Aufgrund der Regelungsmöglichkeit, kann der Operateur bei der Operation so weichteilschonend wie möglich vorgehen. Eine solche Möglichkeit unterstützt eine atraumatische Vorgehensweise bei der Operation. Diese Vorgehensweise wird durch die erfinderische Gefäßklemme ermöglicht, wobei diese den weiteren Vorteil bietet, der in der Zuverlässigkeit der Gefäßklemme liegt, ein Gefäß atraumatisch sicher mechanisch zu verschließen. Die Druckkraft für die verschiebbare Branche I, II am Arbeitsteil der Gefäßklemme wird durch Drehungen des Instrumentengriffes am Betätigungswerkzeug erzeugt. Drehungen des Instrumentengriffes bewirken eine Verschiebung des, über ein Feingewinde angeschlossenen Zug- und Druckelements. Das Zug- und Druckelement wirkt dann über die Hebel des Hebelgetriebes auf die Branche I, II ein. Des Weiteren wird die erfinderische Gefäßklemme nur aus einer geringen Anzahl von übersichtlichen Bauteilen gebildet, wodurch auch die Reinigung und Desinfizierung der Gefäßklemme wesentlich erleichtert wird.

Ein Ausführungsbeispiel der Erfindung ist in den Zeichnungen rein schematisch dargestellt und wird nachfolgend näher beschrieben. Es zeigt
- Figur 1: eine schematische Ansicht einer chirurgischen Gefäßklemme aus dem Stand der Technik, und
- Figur 2: eine parallel klemmende Ausführungsform einer erfindungsgemäßen, Gefäßklemme mit geöffnetem Arbeitsmittel, ebenfalls in schematischer Ansicht, gemäß der vorliegenden Erfindung, und
- Figur 3: eine parallel klemmende Ausführungsform einer erfindungsgemäßen Gefäßklemme mit geschlossenem Arbeitsmittel in perspektivischer Seitenansicht, gemäß der vorliegenden Erfindung.

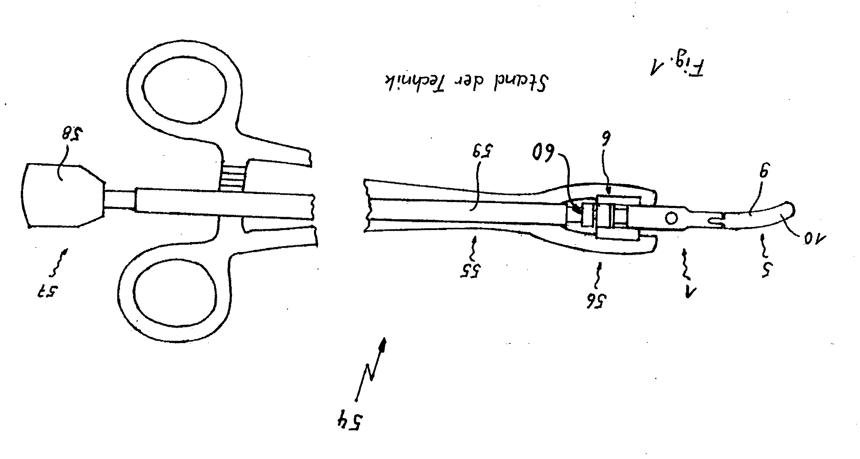

Das in der **Fig.1** in schematischer und prinzipieller Darstellung aufgezeigte chirurgische Instrument aus dem Stand der Technik ist ein, in drei Bauteile zerlegbares chirurgisches Instrument, welches in der EP 2 335 609 B1 offenbart ist. Die drei Bauteile bilden die Basis der Weiterentwicklung. Das dreiteilige chirurgische Instrument kann zwischen der Gefäßklemme **1** und einer Zuführ- und Entnahmevorrichtung **54,** die aus einer Bedieneinrichtung **55** und einem Betätigungswerkzeug **57** besteht, auf einfache Art und Weise wiederholbar getrennt, bzw. zerlegt und komplettiert werden. Die drei Bauteile umfassen ein Betätigungswerkzeug **57** mit einem integrierten Instrumentengriff **58** zum Öffnen und Schließen einer Gefäßklemme **1,** eine Bedieneinrichtung **55** mit einer Greifeinrichtung **56** zum Erfassen einer Gefäßklemme **1** und eine Gefäßklemme **1,** die eine Koppeleinrichtung **6** umfasst, welche von der, an der Bedieneinrichtung **55** angeordneten Greifeinrichtung **56,** erfasst werden kann. Des Weiteren sind an der Gefäßklemme **1** eine Verstelleinrichtung **7,** ein Zug- und Druckelement **4** und eine Arretierungseinrichtung **8** (in **Figur 2** dargestellt) angeordnet, um eine Verstellung der ersten und zweiten Branche I, II **9, 10,** die sich in Relation zur Mittellinie **68** parallel gegenüberliegen, zu ermöglichen. Die Gefäßklemme **1** umfasst ein Arbeitsmittel **5,** bestehend aus der ersten und einer zweiten Branche I, II **9, 10,** welche vom Betätigungswerkzeug **57** geöffnet oder geschlossen werden können. Die vorliegende Erfindung bezieht sich auf eine weitergehende Entwicklung der Gefäßklemme **1**, die einerseits die zwei, parallel zueinander verstellbare Branchen I, II **9, 10** aufweist und die andererseits mit der Bedieneinrichtung **55** und dem Betätigungswerkzeug **57** korrespondieren soll. Diese Weiterentwicklung der erfinderischen Gefäßklemme **1** ist der **Figur 2** zu entnehmen. Die erfinderische chirurgische Gefäßklemme **1** ist also derart zu gestalten, dass dem Chirurgen die, aus dem Stand der Technik bekannte ergonomisch gestaltete Zuführ- und Entnahmevorrichtung **54,** bestehend aus einer Bedieneinrichtung **55** und einem Betätigungswerkzeug **57** mit Instrumentengriff **58,** zur Verstellung der Gefäßklemme **1** für ein vereinfachtes Handling, weiterhin zur Verfügung steht.

Die **Fig.2** zeigt, in schematischer Darstellung, eine parallel klemmende Ausführungsform einer erfindungsgemäßen Gefäßklemme **1** mit geöffnetem Arbeitsmittel **5.** Eine Aufgabe der Erfindung war es, die Übertragung größerer Kräfte vom Instrument der Gefäßklemme **1** auf ein abzuklemmendes Gefäß und eine Öffnung **86** des Arbeitsmittels **5** bereitzustellen, die größer ist als die Öffnung von bekannten Maulteilen "paralleler Klemmen" aus dem Stand der Technik. Als Lösung weist das Arbeitsmittel **5** der Gefäßklemme **1** daher eine variable Öffnung **86** zwischen der ersten und zweiten beweglichen Branche I, II **9, 10** auf, die einen Bereich von 0 mm bis ungefähr 60 mm einnehmen kann. Bezüglich der größeren Kraftübertragung wird auf die nachstehende Beschreibung zu den Ausführungsformen der ersten und zweiten Schwenkarme III, IV **16, 17** und der ersten und zweiten Kniehebel V, VI **24, 25** verwiesen. Die Gefäßklemme **1** wird als zangenförmiges chirurgisches Instrument verwendet, das einen mechanischen Vorgang zwischen seiner ersten und zweiten parallel beabstandeten Branche I, II **9, 10** dazu benutzt, Gewebe oder Gefäße schonend zusammenzudrücken und zu klammern. Das Klammern erfolgt durch den Druck der ersten und zweiten Branche I, II **9, 10,** welcher durch die Drehbewegung des Verstellelementes **11** an der Verstelleinrichtung **7** erzeugt wird. Zur Erzeugung der Drehbewegung an dem Verstellelement **11** wird ein Betätigungswerkzeug **57** mit Instrumentengriff **58,** entsprechend der Ausführung nach **Figur 1****,** eingesetzt, dessen Klinge **60** mit dem Verstellelement **11** in Eingriff gebracht wird. Gemäß der **Figur 1**, wird das Betätigungswerkzeug **57** von einem, an der Bedieneinrichtung **55** angeordneten Hohlzylinder **59,** geführt. Der Hohlzylinder **59** zentriert das Betätigungswerkzeug **57** vor der Verstelleinrichtung **7** und greift mit seiner Klinge **60** in ein Verstellelement **11** der Verstelleinrichtung **7** ein. Die Bedieneinrichtung **55** erfasst mit ihrer Greifeinrichtung **56,** siehe **Figur 1**, die Haltebacken **13** der Koppeleinrichtung **6** einer Gefäßklemme **1.** Nach jeder Drehbewegung am Betätigungswerkzeug **57** ist eine Sperrwirkung für die erste und zweite Branche I, II **9, 10** über die Arretierungseinrichtung **8** vorhanden, um die erste und zweite Branche I, II **9, 10** in jeder Stellung arretieren zu können. Die Drehbewegung am Instrumentengriff **58** führt über die Verstelleinrichtung **7** zu einer translatorischen Verschiebung **12** des Zug- und Druckelementes **4** und über das Hebelgetriebe **2** letztendlich zu einer Verschiebung der ersten und zweiten Branche I, II **9, 10.**

Um eine Verschiebung der parallel angeordneten ersten und zweiten Branche I, II **9, 10** zu realisieren, ist ein erfindungsgemäßes Hebelgetriebe **2** zwischen der Verstelleinrichtung **7** und den parallel verstellbaren ersten und zweiten Branchen I, II **9, 10** angeordnet. Um ein paralleles Klemmen der verstellbaren ersten und zweiten Branche I, II **9, 10** zu ermöglichen, sind zwei Gelenkverbindungen **14, 15,** eine vordere- **14** und eine hintere Gelenkverbindung **15,** vorgesehen. Die vordere Gelenkverbindung **14** und die hintere Gelenkverbindung **15** sind annähernd parallel beabstandet. Die vordere Gelenkverbindung **14** besteht aus einem ersten und einem zweiten Schenkarm III, IV **16, 17,** wobei der erste Schwenkarm III **16** aus einem Paar von Schwenkarmen lila, IIIb **16.1, 16.2** gebildet ist. Die vordere Gelenkverbindung **14** ist über eine erste, zweite und fünfte Gelenkachse IV, V, IV' **18, 19, 20** an der ersten und zweiten Branche I, II **9, 10** und dem Körperelement **3** angelenkt, wobei das Paar von Schwenkarmen.IIIa, IIIb **16.1, 16.2** an seinem einen Ende über die erste Gelenkachse IV **18** drehbeweglich mit der ersten Branche I **9** und an seinem anderen Ende über die zweite Gelenkachse V **19** drehbeweglich mit dem distalen Ende **83** des Körperelements **3** verbunden ist. Die beiden Schwenkarme IIIa, IIIb **16.1, 16.2** sind jeweils außen an der ersten Branche I **9** und außen am Körperelement **3** drehbeweglich befestigt. Dadurch sind die beiden Schwenkarme IIIa, IIIb **16.1, 16.2** parallel beabstandet, wodurch die erste Branche **I 9** und der zweite Schwenkarm IV **17,** beim Schließen des Arbeitsmittels **5,** in den Zwischenraum **21** zwischen die beiden Schwenkarme IIIa, IIIb **16.1, 16.2** eintauchen können. Der zweite Schwenkarm IV **17** hingegen ist an seinem einen Ende über die fünfte Gelenkachse IV' **20** drehbeweglich mit der zweiten Branche II **10** und an seinem anderen Ende über die zweite Gelenkachse V **19** drehbeweglich mit dem distalen Ende **83** des Körperelements **3** verbunden. Die zweite Gelenkachse V **19** ist die gemeinsame Gelenkachse V **19** des ersten Schwenkarmes III **16** und des zweiten Schwenkarmes IV **17** und befindet sich am distalen Ende **83** des Körperelementes **3.** Dieses distale Ende **83** des Körperelementes **3** ist mit einem Gabelkopf F **81** ausgebildet, welcher in einer ersten Nut I **23** den Gelenkkopf G **82** des zweiten Schwenkarmes IV **17** aufnimmt. Der zweite Schwenkarm IV **17** ist, im Gegensatz zum ersten Schwenkarm III **16,** nicht außen an der zweiten Branche II **10** und nicht außen am Körperelement **3** angeordnet, sondern in der zweiten Branche II **10** und im Körperelement **3.** Im Körperelement **3** ist die erste Nut I **23** und in der zweiten Branche **II 10** ist eine Nut **22** eingeformt, in welche der zweite Schwenkarm IV **17** eingreift, wobei der zweite Schwenkarm IV **17** um die fünfte und zweite Gelenkachse IV', V **20, 19** drehbeweglich befestigt ist. Die hintere Gelenkverbindung **15** besteht aus einem ersten und einem zweiten Kniehebel V, VI **24, 25,** wobei die hintere Gelenkverbindung **15** über vier Gelenkachsen III, I, III', I' **26, 27, 28, 29,** eine dritte, vierte, sechste und siebte, an der ersten und zweiten Branche I, II **9, 10** und dem Körperelement **3** angelenkt ist. Der erste Kniehebel V **24** ist an seinem einen Ende über die dritte Gelenkachse III **26** drehbeweglich mit dem proximalen Ende der ersten Branche I **9** und an seinem anderen Ende über die vierte Gelenkachse **I 27** drehbeweglich mit dem Körperelement **3** verbunden. Dieser erste Kniehebel V **24** ist innen an der ersten Branche I **9** und innen am Körperelement **3,** um die dritte und vierte Gelenkachse III, I **26, 27** drehbeweglich befestigt, weil in der ersten Branche I **9** eine Nut **31** und im Körperelement **3** auf der Oberseite **72** eine zweite Nut II **32** eingeformt ist, in welche der erste Kniehebel V **24** eintaucht. Der zweite Kniehebel VI **25** hingegen ist an seinem einen Ende über die sechste Gelenkachse III' **28** drehbeweglich mit der zweiten Branche II **10** und an seinem anderen Ende über die siebte Gelenkachse I' **29** mit dem Körperelement **3** verbunden. Dieser zweite Kniehebel VI **25** ist ebenfalls innen an der zweiten Branche II **10** und innen am Körperelement 3 um die sechste und siebte Gelenkachse III', I' **28, 29** drehbeweglich befestigt, weil in der zweiten Branche II **10** ebenfalls eine Nut **33** und im Körperelement **3** auf der Unterseite **71** eine dritte Nut III **34** eingeformt ist, in welche der zweite Kniehebel VI **25** eintaucht. Die siebte Gelenkachse I' **29** bildet nicht die gemeinsame Gelenkachse vom ersten und zweiten Kniehebel V **24,** VI **25,** weil der erste Kniehebel V **24** eine eigene, von der siebten Gelenkachse I' **29** beabstandete vierte Gelenkachse I **27,** besitzt.

Um eine Verstellung der vorderen und hinteren Gelenkverbindung **14, 15** und somit der ersten und zweiten Branche I, II **9, 10** des Arbeitsmittels **5** vom Betätigungswerkzeug **57** aus, über die Verstelleinrichtung **7** und das Zug-und Druckelement **4** zu bewirken, ist ein Antrieb auf die hintere Gelenkverbindung **15** notwendig. Die hintere Gelenkverbindung **15** besteht aus zwei Kniehebeln V, VI **24, 25,** einem ersten und einem zweiten, deren Ausführungsform identisch ist, wobei der erste und der zweite Kniehebel V, VI **24, 25** symmetrisch um eine Mittellinie **68** des Körperelementes **3** spiegelbildlich angeordnet sind. Es braucht daher nur die technische Ausführungsform des ersten oder des zweiten Kniehebels V, VI **24, 25** betrachtet und beschrieben zu werden. Die Beschreibung erfolgt stellvertretend für beide Kniehebel V, VI **24, 25,** anhand des ersten Kniehebels V **24.** Der erste Kniehebel V **24** weist einen ersten langen- VIIIc **36** und einen ersten kurzen Hebelarm Vlla **35** auf, wobei, wie zuvor beschrieben, der erste Kniehebel V **24** mit dem kurzen ersten Hebelarm Vlla **35** an der vierten Gelenkachse I **27** im Körperelement **3,** angeordnet ist. Der erste lange Hebelarm Vlllc **36** des ersten Kniehebels V **24** weist am freien Ende einen ersten Gelenkkopf A **48** mit Auge auf, während am kurzen ersten Hebelarm Vlla **35** eine erste Feder **I 85** mit zwei Öffnungen, zur Aufnahme von der vierten und achten Gelenkachse I, II, **27, 37,** angeordnet ist. Des Weiteren befindet sich im kurzen ersten Hebelarm Vlla **35** eine weitere achte Gelenkachse II **37.** An dieser achten Gelenkachse II **37** ist das Zug- und Druckelement **4** angeschlossen, wodurch die Verbindung zum Instrumentengriff **58** des Betätigungswerkzeuges hergestellt wird. Somit ist eine durchgehende Verbindung vom Schraub-/Mutter-Antrieb, bestehend aus dem Betätigungswerkzeug **57,** dem Verstellelement **11** und dem Zug- und Druckelement **4,** zum Hebelgetriebe **2** hergestellt, wodurch eine Drehbewegung in eine lineare Verschiebung **12** gewandelt bzw. transferiert wird.

Da der zweite Kniehebel VI **25** spiegelbildlich zum ersten Kniehebel V **24** ausgebildet ist, ist die Verbindung des zweiten Kniehebels VI **25** zum Körperelement **3** analog zum ersten Kniehebel V **24** zu sehen. Der zweite Kniehebel VI **25** weist einen zweiten langen- Vllld **36'** und einen zweiten kurzen Hebelarm Vllb **35'** auf, wobei, wie zuvor beschrieben, der zweite Kniehebel VI **25** und somit der kurze zweite Hebelarm Vllb **35',** an der siebten Gelenkachse I' **29** im Körperelement **3,** angeordnet ist. Gemäß den zuvor aufgezeigten Angaben sind der erste und zweite Kniehebel **24, 25** in der Ausführungsform symmetrisch um die Mittellinie **68** spiegelbildlich angeordnet. D.h., der zweite lange Hebelarm Vllld **36'** des zweiten Kniehebels VI **25** weist am freien Ende einen zweiten Gelenkkopf A' **48'** mit Auge auf, während am zweiten kurzen Hebelarm Vllb **35'** eine zweite Feder II **B5**' mit zwei Öffnungen zur Aufnahme der siebten und einer neunten Gelenkachse I', II' **29, 38,** angeordnet ist. An dieser neunten Gelenkachse II' **38** ist eine Schub-Zug-Stange **39** angeschlossen. Der kurze zweite Hebelarm Vllb **35'** des zweiten Kniehebels VI **25** greift mit seinem Gelenkkopf E **80** in den zweiten Gabelkopf D **79** der Schub-Zug-Stange **39** ein, die um eine gemeinsame neunte Gelenkachse II' **38** drehbar angeordnet ist.

Um eine Verbindung zum Instrumentengriff **58** des Betätigungswerkzeuges **57** herzustellen, ist es notwendig, eine Verbindung zwischen der Schub-Zug-Stange **39** und dem Zug- und Druckelement **4** herzustellen. Erst damit wäre eine durchgehende Verbindung vom Schraub-/Mutter-Antrieb zum kompletten Hebelgetriebe **2** sichergestellt. Um dieses Erfordernis zu erfüllen, weist das Körperelement 3 einen Schlitz **40** auf, welcher das Körperelement **3** durchdringt. Durch diesen Schlitz **40** ist ein Verbindungselement **41** geführt, welches das Zug- und Druckelement **4** mit der Schub-Zug-Stange **39** verbindet. Bei der Verbindung zwischen dem Zug- und Druckelement **4** und der Schub-Zug-Stange **39** handelt es sich um eine feste Verbindung, die nicht lösbar ist. Erst mit dem Verbindungselement **41** besteht eine durchgehende Verbindung vom Instrumentengriff **58** des Betätigungswerkzeuges **57** zu dem zweiten Kniehebel VI **25** des Hebelgetriebes **2.** Dieses ist möglich, weil zwischen dem Zug- und Druckelement **4** und der Schub-Zug-Stange **39** durch das Verbindungselement **41** eine feste Verbindung besteht. Wird das Zug- und Druckelement **4** translatorisch verschoben, verschiebt sich gleichzeitig die Schub-Zug-Stange **39** linear entlang dem Körperelement **3.** Die Schlitzlänge **84** des Schlitzes **40** beträgt daher mindestens die Länge der linearen Verschiebung **12** des Zug- und Druckelementes **4.**

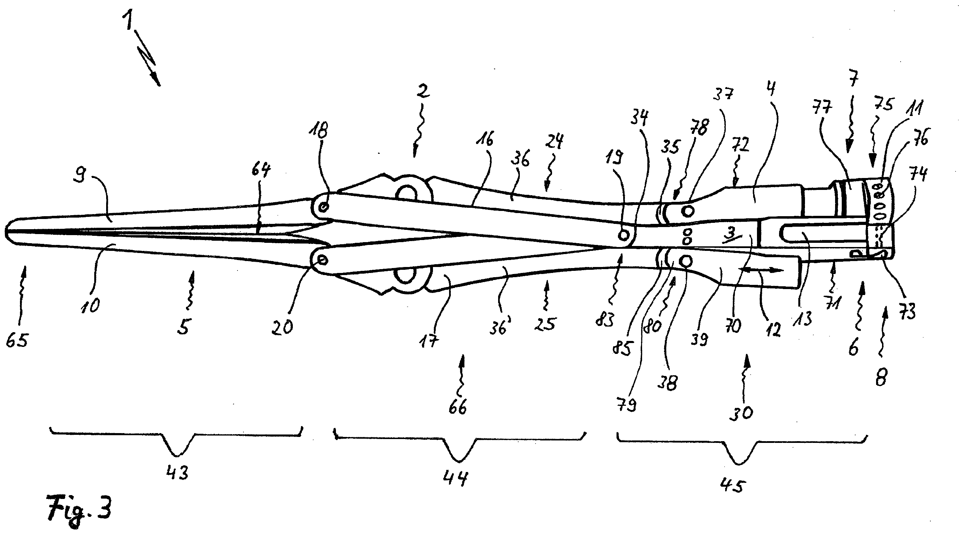

Um ein paralleles Klemmen der verschiebbaren ersten und zweiten Branche I, II **9, 10** zu ermöglichen, ist in der gabelartigen Ausformung, am Ende des Zug- und Druckelementes **4,** der kurze erste Hebelarm Vlla **35** des ersten Kniehebels V **24** über die achte Gelenkachse II **37** angelenkt. Das gleiche gilt für den zweiten Kniehebel VI **25,** dessen kurzer zweite Hebelarm Vllb **35'** in einer gabelartigen Ausformung am Ende der Schub-Zug-Stange **39** über die neunte Gelenkachse II' **38** angelenkt ist. Die achte und neunte Gelenkachse II, II' **37, 38** ist jeweils als Drehlager ausgebildet und wird vom Zug- und Druckelement **4,** sowie von der Schub-Zug-Stange **39** auf einer Länge b, wobei b das Bogenmaß auf einem Kreisbogen **42** ist, verschoben. Aus Übersichtlichkeitsgründen wurde der Kreisbogen **42'** um die neunte Gelenkachse II' **38** nicht eingezeichnet, weil der Kreisbogen **42'** mit dem Kreisbogen **42** identisch ist. Da die Bewegungen der ersten Branche I **9,** des ersten Schwenkarmes III **16,** des ersten Kniehebels V **24** und des Zug- und Druckelementes **4** mit den Bewegungen der zweiten Branche II **10,** des zweiten Schwenkarmes IV **17,** des zweiten Kniehebels VI **25** und der Schub-Zug-Stange **39** identisch sind, erfolgt die Beschreibung des Hebelgetriebes **2** anhand der Bewegungen der ersten Branche I **9,** des ersten Schwenkarmes III **16,** des ersten Kniehebels V **24** und des Zug- und Druckelementes **4.** Die nachstehende Beschreibung zur Funktionsweise und der technischen Ausführung des Hebelgetriebes **2,** wird also mit Hilfe der Hebel der ersten Branche I **9,** des ersten Schwenkarms III **16,** des ersten Kniehebels V **24** und des Zug-und Druckelements **4** vorgenommen, wobei die Beschreibung analog auf die Hebel der zweiten Branche II **10,** des zweiten Schwenkarmes IV **17,** des zweiten Kniehebels VI **25,** und der Schub-Zug-Stange **39** anzuwenden ist. Für die Hebel, den ersten Schwenkarm III **16,** den zweiten Schwenkarm IV **17,** den ersten Kniehebel V **24,** den zweiten Kniehebel VI **25,** das Zug-und Druckelement **4** und die Schub-Zug-Stange **39,** ist die Basis des Hebelgetriebes **2** das Körperelement **3**, an welchem alle Bauteile, einschließlich Verstellelement **7,** Koppeleinrichtung **6** und Arretierungseinrichtung **8,** angeordnet sind. Eine Verschiebung der achten Gelenkachse **37** auf dem Kreisbogen **42** ist aufgrund einer geradlinigen Vorwärts- und Rückwärtsverschiebung **12** des Zug- und Druckelementes **4** alternierend. Des Weiteren ist der kurze erste Hebelarm Vlla **35** des ersten Kniehebels V **24** über eine vierte Gelenkachse I **27** am Körperelement **3** befestigt. Das Körperelement **3** ist in drei Funktionsbereiche I, II, III **43, 44, 45** unterteilt, die in der **Figur 3** näher beschrieben werden. Der kurze erste Hebelarm Vlla **35** des ersten Kniehebels V **24** ist um die vierte Gelenkachse I **27,** welche als Drehlager ausgebildet ist, um einen ersten Winkel a **46** schwenkbar angeordnet. Die Schenkel des ersten Winkels a **46** werden durch das Körperelement **3** und den kurzen ersten Hebelarm Vlla **35** gebildet. Der Winkelbereich des ersten Winkels a **46** wird durch einen Achsabstand I **47,** der sich aus dem Abstand der vierten Gelenkachse I **27** am Körperelement 3 zu der achten Gelenkachse II **37** auf dem kurzen ersten Hebelarm Vlla **35** bzw. dem Zug- und Druckelement **4** ergibt und über die Weglänge s der linearen Verschiebung **12** bestimmt, die sich aus der geradlinigen Verschiebung des Zug- und Druckelementes **4** ergibt. Gleiches bzw. Analoges gilt für den zweiten Winkelbereich α' (der aus Übersichtlichkeitsgründen nicht dargestellt ist). Der Winkelbereich a' ist aber analog zum ersten Winkelbereich **46,** der ebenfalls durch einen Achsabstand zwischen der siebten und neunten Gelenkachse I', II' **29, 38** (nicht dargestellt, der aber analog zum Achsabstand **I 47** ist), bestimmt wird.

Der Achsabstand ergibt sich aus dem Abstand der siebten Gelenkachse I' **29** am Körperelement **3** zu der neunten Gelenkachse II' **38** auf dem kurzen zweiten Hebelarm Vllb **35'** bzw. der Schub-Zug-Stange **39** und wird über die Weglänge s der linearen Verschiebung **12** bestimmt, die sich aus der geradlinigen Verschiebung des Zug- und Druckelementes **4** ergibt.

Der Achsabstand I **47** entspricht dem Radius r vom Drehpunkt der vierten Gelenkachse I **27** bis zum Kreisbogen b, auf welchem sich die achte Gelenkachse II **37** verschiebt. Aus dem Radius r und dem Bogenmaß b, welches annähernd der Weglänge s **12** der linearen Verschiebung **12** des Zug- und Druckelementes **4** entspricht, ergibt sich der Winkel ϑ, der proportional zum ersten Winkel a **46** ist. Der Winkel ϑ ist in der **Figur 2** aus Übersichtlichkeitsgründen nicht dargestellt.

An den kurzen ersten Hebelarm Vlla **35** des ersten Kniehebels V **24** schließt sich der abgeknickte erste lange Hebelarm Vlllc **36** an. Der Winkel zwischen dem ersten kurzen und dem ersten langen Hebelarm Vlla, Vlllc **35, 36** des ersten Kniehebels V **24,** der auch die Funktion eines Gelenkhebels erfüllt, liegt in einem Winkelbereich von ca. 120 Grad bis 150 Grad und beträgt vorzugsweise ca. 135 Grad. Das Zug- und Druckelement **4** und der erste Kniehebel V **24,** welcher vom Zug- und Druckelement **4** um einen bestimmten Winkelbereich verschwenkt werden kann, sind Teil des Hebelgetriebes **2.** Am Ende des ersten Kniehebels V **24** befindet sich der erste Gelenkkopf A **48** mit Auge. Der erste Gelenkkopf A **48** taucht in den ersten Gabelkopf B **49** der verschiebbaren ersten Branche I **9** ein. Der erste Gelenkkopf A **48** und der erste Gabelkopf B **49** sind über die gemeinsame dritte Gelenkachse III **26** drehbeweglich miteinander verbunden. Bei der Verschiebung der achten Gelenkachse II **37,** wird auch gleichzeitig der erste Kniehebel V **24** mit seiner dritten Gelenkachse III **26** verschoben. Gleiches trifft auch analog auf den zweiten Kniehebel VI **25** zu, an dessen Ende sich der zweite Gelenkkopf A' **48'** mit Auge befindet. Der zweite Gelenkkopf A' **48'** taucht in den zweiten Gabelkopf B' **49'** der verschiebbaren zweiten Branche II **10** ein. Der zweite Gelenkkopf A' **48'** und der zweite Gabelkopf B' **49'** sind über die gemeinsame sechste Gelenkachse III' **28** drehbeweglich miteinander verbunden. Die Verschiebung der dritten Gelenkachse III **26** erfolgt ebenfalls auf einem Kreisbogen **50.** Die Größe der Verschiebung der dritten Gelenkachse III **26** auf dem Kreisbogen **50** bzw. auch dessen Begrenzung in der Verschiebung, wird bestimmt durch die Verschiebung der achten Gelenkachse II **37** und das Verschwenken des ersten Kniehebels V **24** um den Drehpunkt der vierten Gelenkachse I **27.** Die Verschiebung des ersten Kniehebels V **24** erfolgt um einen bestimmten dritten Winkel β **51** in einem bestimmten Winkelbereich, der sich aus den Schenkeln des Körperelementes **3** und dem langen ersten Hebelarm Vlllc **36** des ersten Kniehebels V **24** ergibt. Der Winkelbereich liegt in etwa zwischen 5 Grad und 70 Grad und beträgt vorzugsweise ca. 60 Grad. Der Winkelbereich des dritten Winkels β **51** wird durch den Achsabstand II **52,** der sich aus dem Abstand der achten Gelenkachse II **37** zu der dritten Gelenkachse III **26,** auf dem ersten langen Hebelarm Vlllc **36** und dem Achsabstand **I 47** zwischen der achten Gelenkachse **II 37** und der vierten Gelenkachse I **27,** auf dem kurzen ersten Hebelarm VIIIa **35,** sowie durch die Weglänge s der linearen Verschiebung **12,** die sich aus der geradlinigen Verschiebung des Zug- und Druckelementes **4** und der Verschiebung des ersten Kniehebels V **24** um den Winkel ϑ ergibt, bestimmt. Gleiches bzw. Analoges gilt für den vierten Winkelbereich β', (der aus Übersichtlichkeitsgründen nicht dargestellt ist). Der Winkelbereich β' ist aber analog zum dritten Winkelbereich **51,** und ist ebenfalls u.a. durch einen Achsabstand zwischen der sechsten und neunten Gelenkachse III', II' **28, 38** (nicht dargestellt, der aber analog zum Achsabstand IV **52** ist), bestimmt wird. Die verschiebbare erste Branche I **9** weist, beabstandet zur dritten Gelenkachse III **26** über einen bestimmten Achsabstand **III 53,** die erste Gelenkachse IV **18** auf. Diese erste Gelenkachse IV **18** ist die gemeinsame Gelenkachse IV **18** für das, an der ersten Branche I **9** drehbeweglich angeordnete, Paar von Schwenkarmen IIIa, IIIb **16.1, 16.2.** Aufgrund der Seitenansicht, ist nur der vordere Schwenkarm IIIa **16.1** gut sichtbar, während der hintere Schwenkarm IIIb **16.2** größtenteils verdeckt ist. Bei den Schwenkarmen IIIa, IIIb **16.1, 16.2** handelt es sich um ein Paar von Schwenkarmen, die einerseits an den Seitenflächen der ersten Branche I **9** und andererseits an den Seitenflächen des Körperelementes **3** angeordnet sind. D.h., an jeder Seitenfläche der ersten Branche I **9** und des Körperelementes **3** ist ein Schwenkarm IIIa, IIIb **16.1, 16.2,** jeweils durch den Querschnitt der ersten Branche I **9** bzw. des Körperelementes **3** beabstandet, angeordnet. Das obere Ende des ersten Schwenkarmes III **16** ist an der ersten Branche I **9** um die erste Gelenkachse IV **18** und das untere Ende des ersten Schwenkarmes III **16** ist am Körperelement **3** um die zweite Gelenkachse V **19** drehbeweglich angeordnet. Die erste Gelenkachse IV **18** und die zweite Gelenkachse V **19** bilden einen bestimmten Achsabstand IV **61.** Der Achsabstand IV **61** ist derart konstruktiv ausgelegt, dass sich die verschiebbare erste Branche I **9** zur verschiebbaren zweiten Branche II **10** immer in einem, im Wesentlichen parallelen Zustand befindet. Bei der Verschiebung der ersten Branche I **9** durch den ersten Kniehebel V **24,** wird die erste Branche I **9** über die Verbindung des Paares von Schwenkarmen IIa, IIIb **16.1, 16.2** zum Körperelement **3** zwangsgeführt. Aufgrund dieser Zwangsführung durch das Paar von Schwenkarmen IIIa, IIIb **16.1, 16.2,** bleibt die verschiebbare erste Branche I **9** immer in einer parallelen Stellung zur verschiebbaren zweiten Branche II **10.** Um dieses Erfordernis der parallelen Stellung der verschiebbaren ersten Branche I **9** zur verschiebbaren zweiten Branche II **10** in jeder geöffneten und geschlossenen Position erfüllen zu können, muß das Paar von Schwenkarmen IIIa, IIIb **16.1,16.2** um den Drehpunkt der zweiten Gelenkachse V **19** und um den Drehpunkt der ersten Gelenkachse IV **18** drehbeweglich angeordnet sein, wobei die zweite Gelenkachse V **19** einen bestimmten Achsabstand **61** von der, im Körperelement **3** angeordneten vierten Gelenkachse I **27,** aufweist.

Die Verschiebung der dritten Gelenkachse III **26** durch den ersten Kniehebel V **24** bewirkt eine gleichzeitige Verschiebung der ersten Gelenkachse IV **18.** Die Verschiebung der ersten Gelenkachse IV **18** erfolgt ebenfalls auf einem Kreisbogen **62.** Die Größe der Verschiebung der ersten Gelenkachse IV **18** auf dem Kreisbogen **62** bzw. auch dessen Begrenzung in der Verschiebung, wird bestimmt durch die Verschiebung der achten Gelenkachse **II 37** und somit des ersten Kniehebels V **24** und durch die Verschwenkung des ersten Kniehebels V **24** um den Drehpunkt in der vierten Gelenkachse I **27.** Damit eine parallele Verschiebung der ersten Branche I **9** zur zweiten Branche II **10** erfolgen kann, muss der Kreisbogen **62** die gleiche Größe aufweisen wie der Kreisbogen **50.** Die Verschiebung der dritten Gelenkachse III **26** auf dem Kreisbogen **50** und die Verschiebung der ersten Gelenkachse IV **18** auf dem Kreisbogen **62,** muss gleichzeitig und parallel zueinander beabstandet erfolgen. Beide Gelenkachsen IV **18,** III **26,** die erste und die dritte, müssen gleichzeitig den gleichen Weg auf den Kreisbögen **50, 62** zurücklegen, nur dadurch bleibt die parallele Stellung der ersten Branche I **9** zur zweiten Branche II **10** erhalten. Der erste lange Hebelarm Vlllc **36** des ersten Kniehebels V **24** und der kurze erste Hebelarm Vlla **35** sind aber nicht parallel zu dem Paar von Schwenkarmen I IIIa, IIIb **16.1, 16.2** beabstandet. Die gegenüber liegenden Seiten, das Paar von Schwenkarmen I IIIa, IIIb **16.1, 16.2** und der erste Kniehebel V **24** verlaufen daher nicht parallel. Bei dieser Anordnung handelt es sich um ein Antiparallelogramm, mit dem geradlinige Bewegungen in kreisförmige Bewegungen umgewandelt werden. Die erste parallelogrammartige Gelenkverbindung des Hebelgetriebes **2** mit starren Seiten und Gelenken an den Eckpunkten, weist gegenüber einem Parallelogramm mit vier Eckpunkten jedoch fünf Gelenkachsen IV **18,** V **19,** III **26,** I **27,** II **37,** nämlich die erste, zweite, dritte, vierte und achte Gelenkachse, auf, woraus sich auch fünf Achsabstände I **47,** II **52,** III **53,** IV **61,** V **63** ergeben. Der Achsabstand V **63** ergibt sich aus dem Abstand der zweiten Gelenkachse V **19** zu der vierten und siebten Gelenkachse I, I' **27, 29.** Gleiches gilt für die spiegelbildliche zweite parallelogrammartige Gelenkverbindung mit den Gelenkachsen V **19,** IV' **20,** III' **28,** I' **29,** II' **38,** nämlich die zweite, fünfte, sechste, siebte und neunte Gelenkachse des Hebelgetriebes **2,** die auch fünf Achsabstände (nicht dargestellt) aufweisen. Das Hebelgetriebe 2 umfasst in Summe aber nur neun Gelenkachsen IV **18,** V **19,** IV' **20,** III **26,** I **27,** III' **28,** I' **29,** II **37** und II' **38,** nämlich die erste, zweite, dritte, vierte, fünfte, sechste, siebte, achte und neunte Gelenkachse, weil die zweite Gelenkachse V **19** vom ersten Schwenkarm III **16** und vom zweiten Schwenkarm IV **17** gemeinsam genutzt wird.

Fünf Achsabstände I **47,** II **52,** III **53,** IV **61,** V **63** bilden gleichzeitig fünf Seiten, die im weitesten Sinne der typischen Hausform eines Staudenhauses entsprechen. Nur der Achsabstand V **63** zwischen der zweiten Gelenkachse V **19** und der vierten Gelenkachse I **27** und der Achsabstand V **53** zwischen der zweiten Gelenkachse IV **18** und der dritten Gelenkachse III **26** sind gleich, alle anderen Achsabstände I **47,** II **52,** IV **61** sind ungleich. Da sich die zueinander beabstandete erste und zweite Branche I, II **9, 10** aber parallel zueinander nähern (aufeinander zu) oder entfernen sollen (voneinander weg), ist der erfindungsgemäße erste Kniehebel V **24** konstruktiv derart ausgebildet, dass die beiden unterschiedlichen Bewegungen, die der erste Kniehebel V **24** gleichzeitig um seine vierte Gelenkachse I **27** und seine achte Gelenkachse II **37** ausführt, zu einer resultierenden Bewegung führen. Aufgrund dieser resultierenden Bewegung des ersten Kniehebels V **24,** können die zweite und dritte Gelenkachse IV **18,** III **26** eine gleichbleibende Kreisbewegung bzw. Verschiebung auf den Kreisbögen **50, 62** vornehmen. Diese zuvor angegebene Beschreibung, ist analog für die zweite Branche II **10,** den zweiten Schwenkarm IV **17,** den zweiten Kniehebel VI **25,** die Schub-Zug-Stange **39** sowie des Körperelementes **3** anzuwenden.

Die **Fig.3** zeigt eine parallel klemmende Ausführungsform einer erfindungsgemäßen Gefäßklemme **1** mit geschlossenem Arbeitsmittel **5.** Aus der perspektivischen Seitenansicht ist das tragende Körperelement **3** mit den erfindungsgemäßen Ausführungen des Hebelgetriebes **2** ersichtlich. Die in der **Figur 2** aufgezeigten Bezugszeichen werden hier analog übernommen, aber nicht näher beschrieben. Des Weiteren können Bezugszeichen aus der **Figur 3** in der **Figur 2** aufgeführt sein. Grundsätzlich weist die Gefäßklemme **1** drei Funktionsbereiche I, II, III **43, 44, 45** auf. Die drei Funktionsbereiche I, II, III **43, 44, 45** betreffen das Arbeitsmittel **5** im Funktionsbereich I **43,** das Hebelgetriebe **2** im Funktionsbereich II **44** und einen Trägerteil **30** im Funktionsbereich III **45.**

Das Arbeitsmittel **5** aus dem Funktionsbereich I **43** weist; die zwei verschiebbare Branchen I, II **9, 10,** die im geschlossenen Zustand am freien distalen Ende **65** einen ovalen Querschnitt bilden, auf. Das Mittelteil **66** des Funktionsbereiches II **44** weist die Hebelmechanik des Hebelgetriebes **2** auf, bestehend aus zwei Schwenkarmen III, IV **16, 17,** dem ersten und dem zweiten, die über eine erste und eine fünfte Gelenkachse IV, IV' **18, 20** mit der ersten und zweiten Branche I, II **9**, **10** und über eine zweite Gelenkachse V **19** mit dem Körperelement **3** verbunden sind, und bestehend aus einem ersten und einem zweiten Kniehebel V, VI **24, 25,** die über die dritte und sechste Gelenkachse III, III' **26, 28** mit der ersten und zweiten Branche I, II **9, 10** und über die vierte und siebte Gelenkachse I, I' **27, 29** mit dem Körperelement **3** verbunden sind. Das Trägerteil **30** weist ein Zug- und Druckelement **4** und eine Schub-Zug-Stange **39** auf, die mit den ersten und zweiten Kniehebeln V, VI **24, 25** über die achte und neunte Gelenkachse II, II' **37, 38** verbunden sind.

Der erste Funktionsbereich I **43,** der das Arbeitsmittel **5** der Gefäßklemme **1** aufzeigt, wird aus zwei Branchen I, II **9**, **10** gebildet, die jeweils eine Verzahnung **64** aufweisen. Die Verzahnung **64** der ersten und zweiten Branche I, II **9, 10** endet am freien Ende, dem distalen Ende **65** der Gefäßklemme **1.** Die im Funktionsbereich I **43** angeordnete verstellbare erste und zweite Branche I, II **9, 10,** weisen eine Form auf, die zum distalen Ende **65** hin relativ geradlinig und verjüngend verläuft. Die Länge des Funktionsbereiches I **43** beträgt ungefähr 1/3 der Gesamtlänge der Gefäßklemme **1.** Stirnseitig ist das frei stehende distale Ende **65** des annähernd ovalen Querschnitts der ersten und zweiten Branche I, II **9**, **10** abgerundet.

Der zweite Funktionsbereich II **44** besteht aus dem Mittelteil **66,** wobei die Länge des Funktionsbereiches II **44** ungefähr 1/3 der Gesamtlänge der Gefäßklemme **1** beträgt. Der Mittelteil **66** enthält keine Verzahnung **64** mehr, nimmt aber einen Teil der erfindungsgemäßen Hebelmechanik des Hebelgetriebes **2** auf. Die Hebelmechanik im Mittelteil **66** der Gefäßklemme **1** besteht aus dem zweiten Schwenkarm IV **17** und dem ersten angelenkten Schwenkarm III **16.** Das Paar von Schwenkarmen IIIa, IIIb **16.1, 16.2** ist parallel beabstandet und erstrecken sich in die gleiche Richtung wie der zweite Schwenkarm IV **17,** uzw. von der ersten Branche I **9** zum Körperelement **3** hin. Der erste Schwenkarm III **16** bzw. jeder seiner Schwenkarme IIIa, IIIb **16.1, 16.2** ist im Mittelteil **66** der Gefäßklemme **1** an der Seitenfläche des freien distalen Endes **83** des Körperelementes **3** angeordnet, während der zweite Schwenkarm IV **17** am distalen Ende **83** des Körperelementes **3** in einer gabelförmigen Ausnehmung (erste Nut I **23**) drehbeweglich angeordnet ist. Der Mittelteil **66** der Gefäßklemme **1** weist an einer entsprechenden Stelle die zweite Gelenkachse V **19** auf, um welche der erste Schwenkarm III **16** um einen fünften Winkelbereich γ **67** (siehe **Figur 2****)** verschwenkbar sind. Gleiches bzw. Analoges gilt für den sechsten Winkelbereich γ', (der aus Übersichtlichkeitsgründen nicht dargestellt ist). Der Winkelbereich γ' ist aber analog zum fünften Winkelbereich γ **67.** Ist das Arbeitsmittel **5,** wie hier gezeigt, geschlossen, liegen die erste und zweite Branche I, II **9, 10** unmittelbar aufeinander und die freien Enden der ersten und zweiten Branche I, II **9, 10** am distalen Ende **65** sind deckungsgleich. Dieses ist nur möglich, weil das Paar von Schwenkarmen IIIa, IIIb **16.1, 16.2** teilweise einen Teil des zweiten Schwenkarmes IV **17** zwischen sich aufnehmen können. Der fünfte Winkel γ **67** zwischen dem Schenkel des ersten und zweiten Schwenkarmes III, IV **16, 17** und der verlängerten Mittellinie **68** des Körperelementes **3,** beträgt in der geschlossenen Stellung des Arbeitsmittels **5** ungefähr 5 bis 10 Grad, vorzugsweise 8 Grad.

Das andere freie Ende des Körperelementes **3** liegt am proximalen Ende **69** im Funktionsbereich III **45,** der als Trägerteil **30** ausgebildet ist. Die Länge des Funktionsbereiches III **45** beträgt ungefähr 1/3 der Gesamtlänge der Gefäßklemme **1.** Das Trägerteil **30** weist einen, in etwa rechteckigen Querschnitt auf, während der Querschnitt am distalen Ende **65** bei geschlossener erster und zweiter Branche I, II **9**, **10** in etwa quadratisch ist. Der Querschnitt am proximalen Ende **69** des Trägerteils **30** hingegen weist einen rechteckigen Querschnitt auf, der in etwa drei- bis vierfach so groß ist wie der Querschnitt am distalen Ende **65** der ersten und zweiten Branche I, II **9, 10.** Aufgrund des rechteckigen Querschnitts sind vier Seiten mit jeweils einer ebenen Fläche, zwei Seitenflächen **70,** einer Fläche an der Unterseite **71** und einer an der Oberseite **72** vorhanden. Das Trägerteil **30** weist an den zwei parallel beabstandeten Seitenflächen **70** und am proximalen Ende **69** des Körperelementes **3,** jeweils eine der Haltebacken **13** auf. Die beiden parallel beabstandeten und sich gegenüber liegenden Haltebacken **13** sind einstückig mit dem Trägerteil **30** und somit mit dem Körperelement **3** verbunden. Die Haltebacken **13** bilden die Kupplungsmittel der Koppeleinrichtung **6.** Des Weiteren befindet sich im Funktionsbereich III **45** am proximalen Stirnende **69** die Arretierungseinrichtung **8.** Die Arretierungseinrichtung **8** wird aus der Blattfeder **73,** die an der Unterseite **71** des Trägerteils **30** angeordnet ist und dem, in einer Bohrung geführten Arretierungsstift **74,** gebildet, der in das, in das Verstellelement **11** angeordnete Rastprofil **75,** eingreift. Das Rastprofil **75** in dem Verstellelement **11** besteht im Prinzip aus aneinander gereihten, angedeuteten Bohrungen **76** geringer Tiefe bzw. kleiner Zentrierungen.

Auf der Oberseite **72** des Körperelementes **3** befindet sich im Funktionsbereich III **45,** im Anschluss an das Verstellelement **11,** eine Halterung **77,** die einerseits mit dem Trägerteil **30** einstückig verbunden ist und andererseits das Zug- und Druckelement **4** aufnimmt und somit die Verstelleinrichtung **7** bildet. Das Zug-und Druckelement **4** ist am Ende der, der Halterung **77** abgewandten Seite, gabelartig ausgebildet. Der erste Gabelkopf C **78** nimmt das obere Ende des kurzen ersten Hebelarmes Vlla **35** vom ersten Kniehebel V **24** auf, während das untere Ende des kurzen ersten Hebelarmes Vlla **35** in einer, in der Oberseite **72** des Körperelementes **3** eingelassenen zweiten I Nut II **32** (siehe **Figur 2****),** eingreift. Das, im Körperelement **3** eingelassene untere Ende des kurzen ersten Hebelarmes Vlla **35,** ist an einer vierten Gelenkachse **I 27** drehbeweglich befestigt, während das obere Ende des ersten kurzen Hebelarmes Vlla **35** an einer achten Gelenkachse II **37** mit dem Zug- und Druckelement **4** drehbeweglich verbunden ist. Hat das Zug- und Druckelement **4** und somit auch die Schub-Zug-Stange **39** die maximale Weglänge s der linearen Verschiebung **12** in Vorwärtsrichtung zurückgelegt und den ersten und zweiten Kniehebel V, VI **24, 25** vorwärts geschwenkt und vorgeschoben, befinden sich die verschiebbare erste und zweite Branche I, II **9, 10** in ihrer geschlossenen Endstellung. In der Endstellung des ersten und zweiten Kniehebels V, VI **24, 25** beträgt der dritte Winkel β **51** (siehe **Figur 2**) zwischen den Schenkeln der ersten und zweiten langen Hebelarme VIIIc, VIIId **36, 36'** des ersten und zweiten Kniehebels V, VI **24, 25** und der verlängerten Mittellinie **68** des Körperelements **3** ungefähr zwischen 5 bis 8 Grad und die parallel verschiebbare erste und zweite Branche I, II **9, 10** liegen einander an. Hat das Zug- und Druckelement **4** und somit auch die Schub-Zug-Stange **39** die maximale Weglänge s **12** in Rückwärtsrichtung zurückgelegt und den ersten und zweiten Kniehebel V, VI **24, 25** zurück geschwenkt und zurück gezogen, befinden sich die verschiebbare erste und zweite Branche I, II **9, 10** in ihrer fast maximal geöffneten Endstellung, siehe **Figur 2****.** Natürlich können die parallel verschiebbaren Branchen I, II **9, 10** auch jede Position zwischen der maximal geöffneten Endstellung und der geschlossenen Endstellung, zum Klemmen von Gewebe, einnehmen. Jede andere, zwischen den Endstellungen eingenommene Klemmstellung, kann mit Hilfe der Arretierungseinrichtung **8** fixiert werden.

**Bezugszeichenliste**

| | | | |
|---|---|---|---|
| 1 | Gefäßklemme | 30 | Trägerteil |
| 2 | Hebelgetriebe | 31 | Nut (in 9) |
| 3 | Körperelement | 32 | Zweite Nut II (in 3, Oberseite) |
| 4 | Zug-und Druckelement | 33 | Nut (in 10) |
| 5 | Arbeitsmittel | 34 | Dritte Nut III (in 3, Unterseite) |
| 6 | Koppeleinrichtung | 35 | Erster Hebelarm Vlla (kurzer) |
| 7 | Verstelleinrichtung | 35' | Zweiter Hebelarm Vllb (kurzer) |
| 8 | Arretierungseinrichtung | 36 | Erster Hebelarm Vlllc (langer) |
| 9 | Erste Branche I | 36' | Zweiter Hebelarm Vllld (langer) |
| 10 | Zweite Branche II | 37 | Achte Gelenkachse II (in 4, 35) |
| 11 | Verstellelement | 38 | Neunte Gelenkachse II' (39, 35') |
| 12 | Lineare Verschiebung (Weg s) | 39 | Schub-Zug-Stange |
| 13 | Haltebacke | 40 | Schlitz IV (in 3) |
| 14 | Vordere Gelenkverbindung | 41 | Verbindungselement(zw.4, 39) |
| 15 | Hintere Gelenkverbindung | 42 | Kreisbogen(um 37, 38) |
| 16 | Erster Schwenkarm III | 43 | Funktionsbereich I |
| 16.1 | Schwenkarm IIIa (v.16) | 44 | Funktionsbereich II |
| 16.2 | Schwenkarm IIIb (v.16) | 45 | Funktionsbereich III |
| 17 | Zweiter Schwenkarm IV | 46 | Erst. Winkelbereich α (zw.3,35) u.zwei.Winkelbereich.α'(zw.3,35') |
| 18 | Erste Gelenkachse IV (v.9, 16) | | |
| 19 | Zw. Gelenkachse V (v.3,16,17) | 47 | Achsabstand I (v.27, 37) |
| 20 | Fün. Gelenkachse IV' (v.10,17) | 48 | Erster Gelenkkopf A (24, 36) |
| 21 | Zwischenraum | 48' | Zweiter Gelenkkopf A'(25, 36) |
| 22 | Nut (in 10) | 49 | Erster Gabelkopf B (v.9) |
| 23 | Erste Nut I (in 3) | 49' | Zweiter Gabelkopf B'(v.10) |
| 24 | Erster Kniehebel V | 50 | Kreisbogen (um 26, 28) |
| 25 | Zweiter Kniehebel VI | 51 | Dritt. Winkelbereich β (zw.3,24) u.vierterWinkelbereichβ' (zw.3,25) |
| 26 | Dritte Gelenkachse III (v.9, 24) | | |
| 27 | Vierte Gelenkachse I (v.3, 24) | 52 | Achsabstand II (v.26, 37) |
| 28 | Sec. Gelenkachse III' (v.10,25) | 53 | Achsabstand III (v.18, 26) |
| 29 | Siebte Gelenkachse I' (v.3,25) | 54 | Zuführ- u. Entnahmevorrichtung |
| 55 | Bedieneinrichtung | 75 | Rastprofil |
| 56 | Greifeinrichtung | 76 | Bohrung |
| 57 | Betätigungswerkzeug | 77 | Halterung |
| 58 | Instrumentengriff | 78 | Erster Gabelkopf [C] (v.4) |
| 59 | Hohlzylinder | 79 | Zweiter Gabelkopf [D] (v.39) |
| 60 | Klinge | 80 | Gelenkkopf [E](v.25,35') |
| 61 | Achsachstand IV (v.18,19) | 81 | Gabelkopf [F] (v.3) |
| 62 | Kreisbogen (um 18,20) | 82 | Gelenkkopf [G] (v.17) |
| 63 | Achsabstand V (v.19,27) Ende (v.3) | 83 | Distales |
| 64 | Verzahnung | 84 | Schlitzlänge |
| 65 | Distales Ende (v.1) | 85 | Erste Feder [I] (v.35) |
| 66 | Mittelteil | 85' | Zweite Feder [II] (v. 35') |
| 67 | Fünf.Winkelbereich γ (zw.3,16) u.sechst. Winkelber γ' | 86 | Öffnung (v.5) (zw.3,17) |
| 68 | Mittelinie (v.1,3,) | | |
| 69 | Proximales Ende (v.1 u.3) | | |
| 70 | Seitenfläche | | |
| 71 | Unterseite (v.3) | | |
| 72 | Oberseite (v.3) | | |
| 73 | Blattfeder | | |
| 74 | Arretierungsstift | | |

## Patentansprüche

1. Chirurgische Gefäßklemme **(1),** umfassend
ein Körperelement **(3)** mit einem proximalen Ende **(69)** und einem distalen Ende **(83)** und einer Mittellinie **(68),** die zwischen diesen Enden verläuft;
eine erste Branche **(9)** und eine zweite Branche **(10),** die sich, in Relation zur Mittellinie **(68),** parallel gegenüberliegen und jeweils über eine parallelogrammartige Gelenkverbindung mit dem Körperelement **(3)** verbunden sind,
die erste parallelogrammartige Gelenkverbindung, bestehend aus einem ersten Schwenkarm **(16),** der an seinem einen Ende über eine erste Gelenkachse **(18)** drehbeweglich mit der ersten Branche **(9)** verbunden ist und an seinem anderen Ende über eine zweite Gelenkachse **(19)** drehbeweglich mit dem distalen Ende **(83)** des Körperelements **(3)** verbunden ist, und
einem, zum ersten Schwenkarm **(16)** proximal und im Wesentlichen parallel dazu verlaufenden ersten Kniehebel **(24),** der an seinem einen Ende über eine dritte Gelenkachse **(26)** drehbeweglich mit dem proximalen Ende der ersten Branche **(9)** verbunden ist und an seinem anderen Ende über eine vierte Gelenkachse **(27)** drehbeweglich mit dem Körperelement **(3)** verbunden ist,
die zweite parallelogrammartige Gelenkverbindung, bestehend aus einem zweiten Schwenkarm **(17),** der an seinem einen Ende über eine fünfte Gelenkachse **(20)** drehbeweglich mit der zweiten Branche **(10)** verbunden ist, und an seinem anderen Ende über die zweite Gelenkachse **(19)** drehbeweglich mit dem distalen Ende **(83)** des Körperelements **(3)** verbunden ist, und
einem, zum zweiten Schwenkarm **(17)** proximal und im Wesentlichen parallel dazu verlaufenden zweiten Kniehebel **(25),** der an seinem einen Ende über eine sechste Gelenkachse **(28)** drehbeweglich mit dem proximalen Ende der zweiten Branche **(10)** verbunden ist und an seinem anderen Ende über eine siebte Gelenkachse **(29)** drehbeweglich mit dem Körperelement **(3)** verbunden ist,
so dass die zwei Branchen **(9, 10)** relativ zueinander aus einer offenen Stellung in eine geschlossene Stellung parallel bewegbar sind;
die chirurgische Gefäßklemme **(1)** weiterhin umfassend
ein Zug- und Druckelement **(4)** und eine Schub-Zug-Stange **(39),** die sich, in Relation zur Mittellinie **(68),** parallel gegenüberliegen, über ein Verbindungselement **(41)** fest miteinander verbunden sind und parallel zur Mittellinie **(68)** verschiebbar mit dem Körperelement **(3)** verbunden sind, wobei das Zug- und Druckelement **(4)** über eine achte Gelenkachse **(37)** drehbeweglich mit dem ersten Kniehebel **(24)** verbunden ist und die Schub-Zug-Stange **(39)** über eine neunte Gelenkachse **(38)** drehbeweglich mit dem zweiten Kniehebel **(25)** verbunden ist, so dass eine Verschiebung **(12)** des Zug-und Druckelements **(4)** und der Schub-Zug-Stange **(39)** in distaler Richtung ein stufenloses Schließen der Branchen **(9, 10)** bewirkt und eine Verschiebung **(12)** des Zug-Druckelements **(4)** und der Schub-Zug-Stange **(39)** in proximaler Richtung ein stufenloses Öffnen der Branchen **(9, 10)** bewirkt; und
am proximalen Ende **(69)** des Körperelements **(3)** eine Koppeleinrichtung **(6),** bestehend aus zwei Haltebacken **(13)** zur Aufnahme einer lösbaren Zuführ-und Entnahmevorrichtung **(54),** eine Verstelleinrichtung **(7)** zur Aufnahme eines lösbaren Betätigungswerkzeuges **(57),** wobei die Verstelleinrichtung **(7)** bei Betätigung die Verschiebung **(12)** des Zug-und Druckelementes **(4)** und der Schub-Zug-Stange **(39)** und dadurch das Öffnen und Schließen der Branchen **(9, 10)** bewirkt, und
eine Arretierungseinrichtung **(8)** zum Arretieren der Stellung der Branchen **(9, 10);**
wobei der erste Schwenkarm **(16)** aus einem Paar von Schwenkarmen **(16.1, 16.2)** besteht,
wobei das Körperelement **(3)** einen rechteckigen Querschnitt aufweist und das Zug-und Druckelement **(4)** und die Schub-Zug-Stange **(39)** an der Ober-und Unterseite **(72, 71)** des rechteckigen Querschnitts entlang verschiebbar sind,
wobei der erste Kniehebel **(24)** einen abgeknickten Gelenkhebel darstellt, der einen ersten langen Hebelarm **(36)** und einen ersten kurzen Hebelarm **(35)** aufweist,
wobei das Zug-und Druckelement **(4)** einen ersten Gabelkopf **(78)** aufweist, der den ersten kurzen Hebelarm **(35)** des ersten Kniehebels **(24)** aufnimmt und in der die achte Gelenkachse **(37)** angeordnet ist, die den ersten Kniehebel **(24)** über den ersten kurzen Hebelarm **(35)** drehbeweglich mit dem Zug-und Druckelement **(4)** verbindet,
wobei der zweite Kniehebel **(25)** einen abgeknickten Gelenkhebel darstellt, der einen zweiten langen Hebelarm **(36')** und einen zweiten kurzen Hebelarm **(35')** aufweist,
wobei die Schub-Zug-Stange **(39)** einen zweiten Gabelkopf **(79)** aufweist, der den zweiten kurzen Hebelarm **(35')** des zweiten Kniehebels **(25)** aufnimmt und in der die neunte Gelenkachse **(38)** angeordnet ist, die den zweiten Kniehebel **(25)** über den zweiten kurzen Hebelarm **(35')** drehbeweglich mit der Schub-Zug-Stange **(39)** verbindet,
wobei das Körperelement **(3)** am distalen Ende **(83)** einen Gabelkopf **(81)** mit einer ersten Nut **(23)** aufweist, die ein Ende des zweiten Schwenkarms **(17)** aufnimmt und in der die zweite Gelenkachse **(19)** angeordnet ist, die das Ende des zweiten Schwenkarms **(17)** drehbeweglich mit dem Körperelement **(3)** verbindet,
wobei das Körperelement **(3)** auf der Oberseite **(72)** eine zweite Nut **(32)** aufweist, die ein Ende des ersten kurzen Hebelarms **(35)** aufnimmt und in der die vierte Gelenkachse **(27)** angeordnet ist, die das Ende des ersten kurzen Hebelarms **(35)** drehbeweglich mit dem Körperelement **(3)** verbindet,
wobei das Körperelement **(3)** auf der Unterseite **(71)** eine dritte Nut **(34)** aufweist, die ein Ende des zweiten kurzen Hebelarms **(35')** aufnimmt und in der die siebte Gelenkachse **(29)** angeordnet ist, die das Ende des zweiten kurzen Hebelarms **(35')** drehbeweglich mit dem Körperelement **(3)** verbindet,
wobei das Körperelement **(3)** einen von der Oberseite **(72)** zur Unterseite **(71)** durchgehenden Schlitz **(40)** aufweist, der das Verbindungselement **(41)** aufnimmt und führt,
wobei der Schlitz **(40)** eine Schlitzlänge **(84)** entlang der Mittellinie **(68)** aufweist, die mindestens der Weglänge s der Verschiebung **(12)** des Zug- und Druckelementes **(4)** und der Schub-Zug-Stange **(39)** entspricht, und
wobei die vierte Gelenkachse **(27)** und die siebte Gelenkachse **(29)** in Richtung der Mittellinie **(68)** zwischen der zweiten Gelenkachse **(19)** und dem Schlitz **(40)** liegen.

2. Chirurgische Gefäßklemme (1) nach Anspruch 1, wobei die oberen Enden der beiden Schwenkarme **(16.1, 16.2)** an der ersten Gelenkachse **(18)** jeweils außen an der Seitenfläche der ersten Branche **(9),** durch den Querschnitt der ersten Branche **(9)** parallel beabstandet, befestigt sind und die unteren Enden der beiden Schwenkarme **(16.1, 16.2)** an der zweiten Gelenkachse **(19)** jeweils außen an der Seitenfläche des Körperelements **(3),** durch den Querschnitt des Körperelements **(3)** parallel beabstandet, drehbeweglich befestigt sind, wobei zwischen den parallel beabstandeten Schwenkarmen **(16.1, 16.2)** ein Zwischenraum **(21)** besteht, in welchen, beim Schließen der Branchen **(9, 10),** der zweite Schwenkarm **(17)** eintaucht.

3. Chirurgische Gefäßklemme **(1)** nach Anspruch 1, wobei der erste lange Hebelarm **(36)** des ersten Kniehebels **(24)** am freien Ende einen ersten Gelenkkopf A **(48)** mit Auge für die dritte Gelenkachse **(26)** und am freien Ende des ersten kurzen Hebelarms **(35)** eine erste Feder **(85)** mit zwei Öffnungen, zur Aufnahme der vierten- und achten Gelenkachse **(27, 37)** aufweist, wobei die erste Feder **(85)** in die zweite Nut **(32)** des Körperelements **(3)** und in eine Nut des ersten Gabelkopfes C **(78)** des Zug-und Druckelementes **(4)** eingreift und dass der zweite lange Hebelarm **(36')** des zweiten Kniehebels **(25)** am freien Ende einen zweiten Gelenkkopf A' **(48')** mit Auge für die sechste Gelenkachse **(28)** und am freien Ende des zweiten kurzen Hebelarms **(35')** eine zweite Feder **(85')** mit zwei Öffnungen, zur Aufnahme der siebten- und neunten Gelenkachse **(29, 38)** aufweist, wobei die zweite Feder (**85'**) in die dritte Nut **(34)** des Körperelements **(3)** und in eine Nut des Gabelkopfes D **(79)** der Schub-Zug-Stange **(39)** eingreift.

4. Chirurgische Gefäßklemme **(1)** nach Anspruch 1, wobei die lineare Verschiebung **(12)** des Zug- und Druckelementes **(4)** auf einer Weglänge s, eine Winkelverschiebung der dritten- und achten-Gelenkachse **(26, 37)** des ersten Kniehebels (**24**) in einem entsprechenden ersten und dritten Winkelbereich α, β **(46, 51)** und eine Winkelverschiebung der ersten Gelenkachse **(18)** des ersten Schwenkarmes **(16)** mit der beweglichen ersten Branche **(9)** in einem fünften Winkelbereich γ **(67),** zur Folge hat, und dass die lineare Verschiebung **(12)** der Schub-Zug-Stange **(39)** auf einer Weglänge s, eine Winkelverschiebung der sechsten- und neunten- Gelenkachse **(28, 38)** des zweiten Kniehebels **(25)** in einem entsprechenden zweiten und vierten Winkelbereich α', β' und eine Winkelverschiebung der fünften Gelenkachse **(20)** des zweiten Schwenkarmes **(17)** mit der beweglichen zweiten Branche **(10)** in einem sechsten Winkelbereich γ', zur Folge hat.

5. Chirurgische Gefäßklemme **(1)** nach Anspruch 1, wobei die Arretierungseinrichtung **(8)** aus einer Blattfeder **(73)** besteht, die an der Unterseite **(71)** des Körperelementes **(3)** angeordnet ist und einem, in einer Bohrung geführten Arretierungsstift **(74),** der in ein, in einem Verstellelement **(11)** angeordneten Rastprofil **(75)** eingreift, welches aus aneinander gereihten Bohrungen **(76)** besteht.

6. Chirurgische Gefäßklemme **(1)** nach Anspruch 1, wobei eine Kraftübertragung über ein Verstellelement **(11)** der Verstelleinrichtung **(7)** auf ein Schraub-/Muttergetriebe und dadurch mit einem Untersetzungsverhältnis auf das Zug- und Druckelement **(4)** erfolgt, und im Anschluss daran die Kraft auf den ersten kurzen Hebelarm **(35)** des ersten Kniehebels **(24)** des Hebelgetriebes **(2)** und von dort aus in einem Übersetzungsverhältnis auf die erste Branche (**9**) übertragen wird, wobei gleichzeitig die Kraft vom Zug- und Druckelement **(4)** über das Verbindungselement **(41)** auf die Schub-Zug-Stange **(39)** weitergeleitet wird, und im Anschluss daran die Kraft auf den zweiten kurzen Hebelarm **(35')** des zweiten Kniehebels **(25)** des Hebelgetriebes **(2)** und von dort aus in einem Übersetzungsverhältnis auf die zweite Branche **(10)** übertragen wird.

## Claims

1. Surgical vascular clamp **(1)** comprising
a body element **(3)** with a proximal end **(69)** and a distal end **(83)** and a centre line **(68),** which runs between these ends;
a first branch **(9)** and a second branch **(10),** which, in relation to the centre line **(68),** are arranged opposite one another in parallel and are each connected to the body element **(3)** via a parallelogram-like articulation joint,
the first parallelogram-like articulation joint consisting of
a first pivot arm **(16),** which at one end is connected rotatably via a first articulation axis **(18)** to the first branch **(9)** and at its other end is connected rotatably via a second articulation axis **(19)** to the distal end **(83)** of the body element **(3),** and
a first toggle lever **(24),** which is arranged proximally of the first pivot arm **(16)** and runs substantially parallel thereto, and at one end is connected rotatably via a third articulation axis **(26)** to the proximal end of the first branch **(9),** and at its other end is rotatably connected via a fourth articulation axis **(27)** to the body element **(3),**
the second parallelogram-like articulation joint consisting of a second pivot arm **(17),** which at one end is rotatably connected via a fifth articulation axis **(20)** to the second branch **(10)** and at its other end is rotatably connected via the second articulation axis **(19)** to the distal end **(83)** of the body element **(3),** and
a second toggle lever **(25),** which is arranged proximally of the second pivot arm **(17)** and runs substantially parallel thereto, and at one end is connected rotatably via a sixth articulation axis **(28)** to the proximal end of the second branch **(10),** and at its other end is rotatably connected via a seventh articulation axis **(29)** to the body element **(3),**
so that the two branches **(9, 10)** are movable relative to one another in parallel from an open position into a closed position;
the surgical vascular clamp **(1)** further comprising
a push-pull element **(4)** and a push-pull rod **(39),** which, in relation to the centre line **(68),** are arranged opposite one another in parallel, are fixedly connected to one another via a connecting element **(41)** and are connected displaceably to the body element **(3)** parallel to the centre line **(68),** wherein the push-pull element **(4)** is connected rotatably to the first toggle lever **(24)** via an eighth articulation axis **(37)** and the push-pull rod **(39)** is connected rotatably to the second toggle lever **(25)** via a ninth articulation axis **(38),** so that a displacement **(12)** of the push-pull element **(4)** and of the push-pull rod **(39)** in the distal direction effects a continuous closing of the branches **(9, 10),** and a displacement **(12)** of the push-pull element **(4)** and of the push-pull rod **(39)** in the proximal direction effects a continuous opening of the branches **(9, 10);** and
at the proximal end **(69)** of the body element **(3),** a coupling device **(6),** consisting of
two retaining jaws **(13)** for receiving a detachable supply and removal device **(54),**
an adjusting device **(7)** for receiving a detachable actuation tool **(57),** wherein the adjusting device **(7),** when actuated, causes the displacement **(12)** of the push-pull element **(4)** and of the push-pull rod **(39)** and thus the opening and closing of the branches **(9, 10),** and
a locking device **(8)** for locking the position of the branches **(9, 10);**
wherein the first pivot arm **(16)** consists of a pair of pivot arms **(16.1, 16.2),**
wherein the body element **(3)** has a rectangular cross-section and the push-pull element **(4)** and the push-pull rod **(39)** are displaceable along the upper side and underside **(72, 71)** of the rectangular cross-section,
wherein the first toggle lever **(24)** is a bent articulation lever, which has a first long lever arm **(36)** and a first short lever arm **(35),**
wherein the push-pull element **(4)** has a first fork head **(78),** which receives the first short lever arm **(35)** of the first toggle lever **(24)** and in which the eighth articulation axis **(37)** is arranged, which rotatably connects the first toggle lever **(24)** via the first short lever arm **(35)** to the push-pull element **(4),**
wherein the second toggle lever **(25)** is a bent articulation lever, which has a second long lever arm **(36')** and a second short lever arm **(35'),**
wherein the push-pull rod **(39)** has a second fork head **(79),** which receives the second short lever arm **(35')** of the second toggle lever **(25)** and in which the ninth articulation axis **(38)** is arranged, which rotatably connects the second toggle lever **(25)** via the second short lever arm **(35')** to the push-pull rod **(39),**
wherein the body element **(3),** at the distal end **(83),** has a fork head **(81)** with a first groove **(23),** which receives an end of the second pivot arm **(17)** and in which the second articulation axis **(19)** is arranged, which rotatably connects the end of the second pivot arm **(17)** to the body element **(3),**
wherein the body element **(3),** on the upper side **(72),** has a second groove **(32),** which receives an end of the first short lever arm **(35)** and in which the fourth articulation axis **(27)** is arranged, which rotatably connects the end of the first short lever arm **(35)** to the body element **(3),**
wherein the body element **(3)** on the underside **(71),** has a third groove **(34),** which receives an end of the second short lever arm **(35')** and in which the seventh articulation axis **(29)** is arranged, which rotatably connects the end of the second short lever arm **(35')** to the body element **(3),**
wherein the body element **(3)** has a slot **(40)** which passes from the upper side **(72)** to the underside **(71)** and which receives and guides the connecting element **(41),**
wherein the slot **(40)** has a slot length **(84)** along the centre line **(68)** which corresponds at least to the path length s of the displacement **(12)** of the push-pull element **(4)** and the push-pull rod **(39),** and
wherein the fourth articulation axis **(27)** and the seventh articulation axis **(29)** lie in the direction of the centre line **(68)** between the second articulation axis **(19)** and the slot **(40).**

2. Surgical vascular clamp **(1)** according to claim 1, wherein the upper ends of the two pivot arms **(16.1, 16.2)** are each secured at the first articulation axis **(18)** externally to the side face of the first branch **(9),** distanced in parallel by the cross-section of the first branch **(9),** and the lower ends of the two pivot arms **(16.1, 16.2)** are each secured rotatably at the second articulation axis **(19)** externally to the side face of the body element **(3),** distanced in parallel by the cross-section of the body element **(3),** wherein there is a gap **(21)** between the pivot arms **(16.1, 16.2)** distanced in parallel, and the second pivot arm **(17)** dips into the gap when the branches **(9, 10)** are closed.

3. Surgical vascular clamp **(1)** according to claim 1, wherein the first long lever arm **(36)** of the first toggle lever **(24),** at the free end, has a first articulation head A **(48)** with an eye for the third articulation axis **(26)** and, at the free end of the first short lever arm **(35),** has a first spring **(85)** with two openings for receiving the fourth and eighth articulation axis **(27, 37),** wherein the first spring **(85)** engages in the second groove **(32)** of the body element **(3)** and engages in a groove of the first fork head C **(78)** of the push-pull element **(4),** and wherein the second long lever arm **(36')** of the second toggle lever **(25)** has, at the free end, a second articulation head A' **(48')** with an eye for the sixth articulation axis **(28)** and, at the free end of the second short lever arm **(35'),** has a second spring **(85')** with two openings for receiving the seventh and ninth articulation axis **(29, 38),** wherein the second spring **(85')** engages in the third groove **(34)** of the body element **(3)** and in a groove of the fork head D **(79)** of the push-pull rod **(39).**

4. Surgical vascular clamp **(1)** according to claim 1, wherein the linear displacement **(12)** of the push-pull element **(4)** along a path length s results in an angular displacement of the third and eighth articulation axis **(26, 37)** of the first toggle lever **(24)** in a corresponding first and third angular range α, β **(46, 51)** and in an angular displacement of the first articulation axis **(18)** of the first pivot arm **(16)** with the movable first branch **(9)** in a fifth angular range γ **(67),** and wherein the linear displacement **(12)** of the push-pull rod **(39)** over a path length s results in an angular displacement of the sixth and ninth articulation axis **(28, 38)** of the second toggle lever **(25)** in a corresponding second and fourth angular range α', β' and in an angular displacement of the fifth articulation axis **(20)** of the second pivot arm **(17)** with the movable second branch **(10)** in a sixth angular range γ'.

5. Surgical vascular clamp **(1)** according to claim 1, wherein the locking device **(8)** consists of a leaf spring **(73),** which is arranged on the underside **(71)** of the body element **(3),** and of a locking pin **(74),** which is guided in a bore and which engages in a detent profile **(75),** which is arranged in an adjusting element **(11)** and which consists of juxtaposed bores **(76).**

6. Surgical vascular clamp **(1)** according to claim 1, wherein force is transferred via an adjusting element **(11)** of the adjusting device **(7)** to a screw/nut drive and thus with a reduction ratio to the push-pull element **(4),** and then the force is transferred to the first short lever arm **(35)** of the first toggle lever **(24)** of the lever gear **(2)** and from there is transferred at a transmission ratio to the first branch **(9),** wherein at the same time the force from the push-pull element **(4)** is forwarded via the connecting element **(41)** to the push-pull rod **(39),** and then the force is transferred to the second short lever arm **(35')** of the second toggle lever **(25)** of the lever gear **(2)** and from there is transferred at a transmission ratio to the second branch **(10).**

## Revendications

1. Pince hémostatique chirurgicale **(1),** comprenant
un élément de corps **(3)** avec une extrémité proximale **(69)** et une extrémité distale **(83)** et une ligne médiane **(68)** qui passe entre ces extrémités ;
une première branche **(9)** et une seconde branche **(10),** qui sont parallèlement opposées par rapport à la ligne médiane **(68)** et sont respectivement reliées à l'élément de corps **(3)** par une liaison articulée en forme de parallélogramme,
la première liaison articulée en forme de parallélogramme, se composant
d'un premier bras pivotant **(16)** qui est relié en mobilité de rotation avec la première branche **(9)** par un premier axe d'articulation **(18)** à une extrémité et en mobilité de rotation avec l'extrémité distale **(83)** de l'élément de corps **(3)** par un deuxième axe d'articulation **(19)** à son autre extrémité, et
un premier levier à rotule **(24)** proximal au premier bras pivotant **(16)** et essentiellement parallèle à celui-ci, qui est relié en mobilité de rotation avec l'extrémité proximale de la première branche **(9)** par un troisième axe d'articulation **(26)** à une extrémité et relié en mobilité de rotation avec l'élément de corps **(3)** par un quatrième axe d'articulation **(27)** à son autre extrémité,
la seconde liaison articulée en forme de parallélogramme se composant
d'un second bras pivotant **(17)** qui est relié en mobilité de rotation avec la deuxième branche **(10)** par un cinquième axe d'articulation **(20)** à une extrémité, et en mobilité de rotation avec l'extrémité distale **(83)** de l'élément de corps **(3)** par le deuxième axe d'articulation **(19)** à son autre extrémité, et
un second levier à rotule **(25)** proximal au second bras pivotant **(17)** et essentiellement parallèle à celui-ci, qui est relié en mobilité de rotation avec l'extrémité proximale de la deuxième branche **(10)** par un sixième axe d'articulation **(28)** à une extrémité et relié en mobilité de rotation avec l'élément de corps **(3)** par un septième axe d'articulation **(29)** à son autre extrémité,
de sorte que les deux branches **(9, 10)** sont mobiles parallèlement entre elles d'une position ouverte à une position fermée ;
la pince hémostatique chirurgicale **(1),** comprenant en outre
un élément de traction et de pression **(4)** et une barre de poussée-traction **(39),** qui sont parallèlement opposées par rapport à la ligne médiane **(68),** sont reliées fixement entre elles par un élément de liaison **(41)** et sont reliées à l'élément de corps **(3)** de manière mobile parallèlement à la ligne médiane **(68),** dans lequel l'élément de traction et de pression **(4)** est relié en mobilité de rotation au premier levier à rotule **(24)** par un huitième axe d'articulation **(37)** et la barre de poussée-traction **(39)** est reliée en mobilité de rotation au second levier à rotule **(25)** par un neuvième axe d'articulation **(38),** de sorte qu'un déplacement **(12)** de l'élément de traction et de pression **(4)** et de la barre de poussée-traction **(39)** dans la direction distale a pour effet une fermeture continue des branches **(9, 10)** et qu'un déplacement **(12)** de l'élément de traction et de pression **(4)** et de la barre de poussée-traction **(39)** dans la direction proximale a pour effet une ouverture continue des branches **(9, 10)** ; et
un dispositif de couplage **(6)** sur l'extrémité proximale **(69)** de l'élément de corps **(3),** se composant
de deux mâchoires de maintien **(13)** pour recevoir un dispositif d'amenée et de prélèvement **(54)** séparable,
un dispositif de décalage **(7)** pour recevoir un outil d'actionnement **(57)** séparable, dans lequel le dispositif de décalage **(7),** en cas d'actionnement, effectue le déplacement **(12)** de l'élément de traction et de pression **(4)** et de la barre de poussée-traction **(39)** et ainsi l'ouverture et la fermeture des branches **(9, 10),** et
un dispositif de blocage **(8)** pour bloquer la position des branches **(9, 10) ;**
dans lequel le premier bras pivotant **(16)** est composé d'une paire de bras pivotants (**16.1, 16.2**),
dans lequel l'élément de corps **(3)** présente une section transversale rectangulaire et l'élément de traction et de pression **(4)** et la barre de poussée-traction **(39)** sont mobiles le long de la face supérieure et inférieure **(72, 71)** de la section transversale rectangulaire,
dans lequel le premier levier à rotule **(24)** représente un levier à articulation plié qui présente un premier bras de levier long **(36)** et un premier bras de levier court **(35),**
dans lequel l'élément de traction et de pression **(4)** présente une première tête de fourche **(78)** qui reçoit le premier bras de levier court **(35)** du premier levier à rotule **(24)** et dans laquelle le huitième axe d'articulation **(37)** est disposé, qui relie le premier levier à rotule **(24)** en mobilité de rotation avec l'élément de traction et de pression **(4)** par le premier bras de levier court **(35),**
dans lequel le second levier à rotule **(25)** est un levier à articulation plié qui présente un second bras de levier long **(36')** et un second bras de levier court (**35'**),
dans lequel la barre de poussée-traction **(39)** présente une seconde tête de fourche **(79)** qui reçoit le second bras de levier court **(35')** du second levier à rotule **(25)** et dans laquelle le neuvième axe d'articulation **(38)** est disposé, qui relie le second levier à rotule **(25)** en mobilité de rotation avec la barre de poussée-traction **(39)** par le second bras de levier court (**35'**),
dans lequel l'élément de corps **(3)** présente sur l'extrémité distale **(83)** une tête de fourche **(81)** avec une première rainure **(23)** qui reçoit une extrémité du second bras pivotant **(17)** et dans laquelle le deuxième axe d'articulation **(19)** est disposé, qui relie l'extrémité du second bras pivotant **(17)** en mobilité de rotation avec l'élément de corps **(3),**
dans lequel l'élément de corps **(3)** présente sur la face supérieure **(72)** une deuxième rainure **(32)** qui reçoit une extrémité du premier bras de levier court **(35)** et dans laquelle le quatrième axe d'articulation **(27)** est disposé, qui relie l'extrémité du premier bras de levier court **(35)** en mobilité de rotation avec l'élément de corps **(3),**
dans lequel l'élément de corps **(3)** présente sur la face inférieure **(71)** une troisième rainure **(34)** qui reçoit une extrémité du second bras de levier court (**35'**) et dans laquelle le septième axe d'articulation **(29)** est disposé, qui relie l'extrémité du second bras de levier court (**35'**) en mobilité de rotation avec l'élément de corps **(3),**
dans lequel l'élément de corps **(3)** présente une fente **(40)** traversante de la face supérieure **(72)** à la face inférieure **(71)** qui reçoit et guide l'élément de liaison **(41),**
dans lequel la fente **(40)** présente une longueur de fente **(84)** le long de la ligne médiane **(68)** qui correspond au moins à la longueur de trajet s du déplacement **(12)** de l'élément de traction et de pression **(4)** et de la barre de poussée-traction **(39),** et
dans lequel le quatrième axe d'articulation **(27)** et le septième axe d'articulation **(29),** dans le sens de la ligne médiane **(68),** reposent entre le deuxième axe d'articulation **(19)** et la fente **(40).**

2. Pince hémostatique chirurgicale **(1)** selon la revendication 1, dans laquelle les extrémités supérieures des deux bras pivotants **(16.1, 16.2)** sont fixées sur le premier axe d'articulation **(18)** respectivement à l'extérieur sur la face latérale de la première branche **(9),** éloignées parallèlement par la section transversale de la première branche **(9)** et les extrémités inférieures des deux bras pivotants **(16.1, 16.2)** sont fixées en mobilité de rotation sur le deuxième axe d'articulation **(19)** respectivement à l'extérieur sur la face latérale de l'élément de corps **(3),** éloignées parallèlement par la section transversale de l'élément de corps **(3),** dans lequel il y a un espace intermédiaire **(21)** entre les bras pivotants **(16.1, 16.2)** éloignés parallèlement, dans lequel plonge le second bras pivotant **(17)** lors de la fermeture des branches **(9, 10).**

3. Pince hémostatique chirurgicale **(1)** selon la revendication 1, dans laquelle le premier bras de levier long **(36)** du premier levier à rotule **(24)** présente sur l'extrémité libre une première tête articulée A **(48)** à œillet pour le troisième axe d'articulation **(26)** et à l'extrémité libre du premier bras de levier court **(35),** un premier ressort **(85)** avec deux ouvertures, pour recevoir les quatrième et huitième axe d'articulation **(27, 37),** dans laquelle le premier ressort **(85)** se met en prise dans la seconde rainure **(32)** de l'élément de corps **(3)** et dans une rainure de la première tête de fourche C **(78)** de l'élément de traction et de pression **(4)** et que le second bras de levier long **(36')** du second levier à rotule **(25)** présente sur l'extrémité libre une seconde tête articulée A' **(48')** à œillet pour le sixième axe d'articulation **(28)** et à l'extrémité libre du second bras de levier court **(35'),** un second ressort (**85'**) avec deux ouvertures, pour recevoir les septième et neuvième axes d'articulation **(29, 38),** dans lequel le second ressort **(85')** se met en prise dans la troisième rainure **(34)** de l'élément de corps **(3)** et dans une rainure de la tête de fourche D **(79)** de la barre de poussée-traction **(39).**

4. Pince hémostatique chirurgicale **(1)** selon la revendication 1, dans laquelle le déplacement linéaire **(12)** de l'élément de traction et de pression **(4)** sur une longueur de trajet s, a pour conséquence un décalage d'angle du troisième et huitième axe d'articulation **(26, 37)** du premier levier à rotule **(24)** dans une première et troisième plage d'angle α, β **(46, 51)** correspondante et un décalage d'angle du premier axe d'articulation **(18)** du premier bras pivotant **(16)** avec la première branche mobile **(9)** dans une cinquième plage d'angle γ **(67),** et que le déplacement linéaire **(12)** de la barre de poussée-traction **(39)** sur une longueur de trajet s, a pour conséquence un décalage d'angle du sixième et neuvième axe d'articulation **(28, 38)** du second levier à rotule **(25)** dans une deuxième et quatrième plage d'angle α', β' correspondante et un décalage d'angle du cinquième axe d'articulation **(20)** du second bras pivotant **(17)** avec la deuxième branche mobile **(10)** dans une sixième plage d'angle γ'.

5. Pince hémostatique chirurgicale **(1)** selon la revendication 1, dans laquelle le dispositif de blocage **(8)** est composé d'un ressort à lames **(73)** qui est disposé sur la face inférieure **(71)** de l'élément de corps **(3)** et d'une tige de blocage **(74)** dirigée dans un perçage qui se met en prise dans un profilé d'encliquetage **(75)** disposé dans un élément de décalage **(11),** lequel est composé de perçages **(76)** rangés les uns contre les autres.

6. Pince hémostatique chirurgicale **(1)** selon la revendication 1, dans laquelle une transmission de force a lieu par un élément de décalage **(11)** du dispositif de décalage **(7)** sur un entraînement vis/écrou et ainsi sur l'élément de traction et de pression **(4)** avec un rapport de démultiplication et suite à cela, la force est transmise sur le premier bras de levier court **(35)** du premier levier à rotule **(24)** de l'entraînement de levier **(2)** et de là sur la première branche **(9)** dans un rapport de translation, dans laquelle parallèlement, la force est transmise de l'élément de traction et de pression **(4)** à la barre de poussée-traction **(39)** par le biais de l'élément de liaison **(41)** et suite à cela, la force est transmise sur le second bras de levier court **(35')** du second levier à rotule **(25)** de l'entraînement de levier **(2)** et de là sur la deuxième branche **(10)** dans un rapport de translation.
